# EUROPÄISCHE PATENTSCHRIFT

(11) **EP 2 064 253 B1**
(45) Veröffentlichungstag und Bekanntmachung des Hinweises auf die Patenterteilung: **18.11.2009**
(21) Anmeldenummer: 07820228.0
(22) Anmeldetag: 14.09.2007
(51) Int. Cl.: C08F 220/18, C08F 226/00, C08F 220/04, A61K 8/81

(54) **AMPHOLYTISCHES COPOLYMER AUF BASIS QUATERNISIERTER STICKSTOFFHALTIGER MONOMERE**
AMPHOLYTIC COPOLYMER BASED ON QUATERNIZED NITROGEN-CONTAINING MONOMERS
COPOLYMÈRE AMPHOLYTIQUE À BASE DE MONOMÈRES CONTENANT DE L'AZOTE QUATERNISÉ

(30) Priorität: 15.09.2006 EP 06120778; 29.06.2007 EP 07111467
(43) Veröffentlichungstag der Anmeldung: 03.06.2009
(73) Patentinhaber: BASF SE, 67056 Ludwigshafen (DE)
(72) Erfinder: NGUYEN-KIM, Son, 69502 Hemsbach (DE); JENTZSCH, Axel, 67063 Ludwigshafen (DE); BOUILLO, Nathalie, 76532 Baden-Baden (DE)
(86) Internationale Anmeldenummer: PCT/EP2007/059725
(87) Internationale Veröffentlichungsnummer: WO 2008/031892

(56) Entgegenhaltungen:
- WO-A-01/62809
- WO-A-2005/058988

## Beschreibung

Die vorliegende Erfindung betrifft ein ampholytisches Copolymer auf Basis quaternisierter stickstoffhaltiger Monomere, das einen molaren Überschuss an kationogenen/kationischen Gruppen gegenüber anionogenen/anionischen Gruppen aufweist, kosmetische oder pharmazeutische Mittel, die wenigstens ein solches ampholytisches Copolymer enthalten sowie weitere Verwendungen dieser Copolymere.

Polymere mit einer größeren Anzahl ionisch dissoziierbarer Gruppen in der Hauptkette und/oder einer Seitenkette werden als Polyelektrolyte bezeichnet. Weisen diese Polymere sowohl anionogene/anionische als auch kationogene/kationische Gruppen auf, so handelt es sich um amphotere Polyelektrolyte bzw. ampholytische Polymere. Ampholytische Polymere mit ausreichender Anzahl dissoziierbarer Gruppen sind wasserlöslich oder wasserdispergierbar und haben vielfältige Anwendungen im Bereich der Anstrichmittel, Papierhilfsmittel, Hygienemittel, bei der Textilherstellung sowie speziell in der Pharmazie und Kosmetik gefunden.

Kosmetisch und pharmazeutisch akzeptable wasserlösliche Polymere dienen beispielsweise in Seifen, Cremes und Lotionen als Formulierungsmittel, z. B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik besteht darin, die Eigenschaften des Haares zu beeinflussen. In der Pharmazie dienen sie beispielsweise als Beschichtungsmittel oder Bindemittel für feste Arzneiformen. Für die Haarkosmetik werden filmbildende Polymere mit ionischen Gruppen beispielsweise als Conditioner dazu eingesetzt, um die Trocken- und Nasskämmbarkeit, das Anfassgefühl, den Glanz und die Erscheinungsform des Haars zu verbessern sowie dem Haar antistatische Eigenschaften zu verleihen. Je nach Anwendungszweck werden dabei wasserlösliche Polymere mit kationischen oder anionischen Funktionalitäten eingesetzt. So weisen Polymere mit kationischen funktionellen Gruppen strukturell bedingt eine hohe Affinität zur negativ geladenen Oberfläche des Haares auf. Polymere mit anionischen Funktionalitäten, wie z. B. gegebenenfalls vernetzte Polyacrylsäure, dienen beispielsweise als Verdicker, weiterhin werden carboxylatgruppenhaltige Polymere beispielsweise zur Festigung von Haarfrisuren eingesetzt.

Für die Haarkosmetik werden filmbildende Polymere weiterhin als Festigerharze eingesetzt, um der Frisur Halt zu verleihen. Anforderungen an Festigerharze sind zum Beispiel eine starke Festigung bei hoher Luftfeuchtigkeit, Elastizität, Auswaschbarkeit vom Haar, Verträglichkeit in der Formulierung und ein angenehmer Griff des damit behandelten Haares. Zur Festigung von Haarfrisuren werden beispielsweise Vinyllactam-Homo- und Copolymere und carboxylatgruppenhaltige Polymere eingesetzt.

Schwierigkeiten bereitet oft die Bereitstellung von Produkten mit einem komplexen Eigenschaftsprofil. So besteht ein Bedarf an Polymeren für haarkosmetische Mittel, die zur Bildung im Wesentlichen glatter, klebfreier Filme befähigt sind, die gegenüber dem Haar eine gute Festigungswirkung (auch bei hoher Luftfeuchtigkeit) aufweisen und dem Haar gleichzeitig gute sensorisch erfassbare Eigenschaften, wie Elastizität und einen angenehmen Griff, verleihen. Sollen diese Polymere in Haarsprayformulierungen eingesetzt werden, so ist zudem eine gute Treibgasverträglichkeit, die Eignung für einen Einsatz in low-VOC-Formulierungen, eine gute Löslichkeit in Wasser oder wässrig/alkoholischen Lösungsmittelgemischen und eine gute Auswaschbarkeit erwünscht.

In vielen Fällen lässt sich das gewünschte Eigenschaftsprofil nur durch Einsatz mehrerer kosmetisch aktiver Komponenten, beispielsweise mehrerer Polymere mit ionischen Gruppen erzielen. Dabei zeigt sich jedoch häufig eine Unverträglichkeit der verschiedenen Komponenten miteinander, was beispielsweise dazu führen kann, dass sich keine klaren Formulierungen mehr herstellen lassen. Der Einsatz mehrerer miteinander nicht ausreichend verträglicher Polyelektrolyte kann zu einem unerwünschten Aussalzen führen. Es besteht daher Bedarf an kosmetisch und pharmazeutisch verträglichen Polyelektrolyten, die bei einem Einsatz als einzige Polymerkomponente geeignet sind, ein bestimmtes Eigenschaftsprofil bereitzustellen und/oder die mit einer Vielzahl verschiedener Komponenten verträglich sind.

Die US 4,358,567 beschreibt Haarpolymere mit Betain-Struktur, die durch Umsetzung eines Copolymers auf Basis von Aminoalkyl(meth)acrylaten mit Natrium- oder Kaliummonochloracetat erhältlich sind.

Die EP-A-0 330 174 beschreibt eine Haarfestigergelzusammensetzung, die ein teilweise oder vollständig neutralisiertes Salz eines vernetzten carboxylgruppenhaltigen Polymers, ein amphoteres Harz und ein Lösungsmittel enthält. Bei dem amphoteren Harz kann es sich um Copolymere mit Betain-Struktureinheiten oder Copolymere, die durch Copolymerisation wenigstens eines Monomers mit sauren Gruppen und wenigstens eines Monomers mit basischen Gruppen erhältlich sind, handeln.

Die GB-A 2,088,209 beschreibt ein Haarbehandlungsmittel auf der Basis von amphoteren Polymeren und anionischen Polymeren. Das amphotere Polymer kann dabei Monomereinheiten enthalten, die sich von mit Dimethylsulfat oder Diethylsulfalt quaternisiertem Dimethylaminoethylmethacrylat ableiten.

Die WO 01/62809 beschreibt ein kosmetisches Mittel, das wenigstens ein wasserlösliches oder wasserdispergierbares Polymer enthält, das
a) 5 bis 50 Gew.-% wenigstens eines α,β-ethylenisch ungesättigten Monomers mit einer tert.-Butylgruppe,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül, und
d) 0 bis 30 Gew.-% wenigstens einer weiteren α,β-ethylenisch ungesättigten Verbindung, wobei es sich um Verbindungen mit mindestens einer anionogenen und/oder anionischen Gruppe pro Molekül handeln kann,
eingebaut enthält.

Die US 3,927,199 beschreibt eine Haarfestigerzusammensetzung, die ein filmbildendes Binderharz auf Basis eines Copolymers enthält, das 1) N-Alkylacrylamide oder -methacrylamide, 2) säuregruppenhaltige Monomere und 3) wenigstens ein weiteres Comonomer einpolymerisiert enthält.

Die US 4,237,253 beschreibt Copolymere für Haarbehandlungsmittel, die 22 bis 64 Mol-% N,N-Dimethylamino-2-ethylmethacrylat, 13 bis 71 Mol-% Methylmethacrylat, 6 bis 23 Mol-% Methacrylsäure und bis zu 22 Mol-% weitere Monomere einpolymerisiert enthalten.

Die WO 95/35087 beschreibt ein amphoteres Haarfestigerpolymer für die Verwendung in Haarsprays und Gelen, das 40 bis 90 Gew.-% eines hydroxylgruppenhaltigen Monomers, 1 bis 20 Gew.-% eines säuregruppenhaltigen Monomers und 1 bis 20 Gew.-% eines amingruppenhaltigen Monomers einpolymerisiert enthält.

Die WO 2004/058837 beschreibt ein ampholytisches Copolymer, das durch radikalische Copolymerisation von
a) wenigstens einer ethylenisch ungesättigten Verbindung mit mindestens einer anionogenen und/oder anionischen Gruppe,
b) wenigstens einer ethylenisch ungesättigten Verbindung mit mindestens einer kationogenen und/oder kationischen Gruppe,
c) wenigstens einer ungesättigten amidgruppenhaltigen Verbindung
sowie gegebenenfalls weiterer Comonomere erhältlich ist. Beschrieben sind weiterhin Polyelektrolyt-Komplexe, die ein solches ampholytisches Copolymer enthalten sowie kosmetische oder pharmazeutische Mittel auf Basis dieser ampholytischen Copolymere und Polyelektrolyt-Komplexe.

Die WO 2004/022616 beschreibt die Verwendung von Polymeren, die erhältlich sind durch
(i) radikalisch initiierte Copolymerisation von Monomergemischen aus
   (a) mindestens einem kationischen Monomeren oder quaternisierbaren Monomeren,
   (b) gegebenenfalls einem wasserlöslichen Monomeren,
   (c) gegebenenfalls einem weiteren radikalisch copolymerisierbaren Monomeren,
   (d) mindestens einem als Vernetzer wirkenden Monomeren mit mindestens zwei ethylenisch ungesättigten, nichtkonjugierten Doppelbindungen, und
   (e) mindestens einem Regler,
(ii) anschließende Quaternisierung oder Protonierung des Polymeren, sofern als Monomeres (a) ein nicht oder nur partiell quaternisiertes Monomer eingesetzt wird,
in haarkosmetischen Zubereitungen.

Die WO 2005/005497 beschreibt eine wässrige Polymerdispersion Pd), die erhältlich ist durch radikalische Polymerisation eines Monomergemischs M), enthaltend
a) wenigstens eine α,β-ethylenisch ungesättigte amidgruppenhaltige Verbindung der allgemeinen Formel I 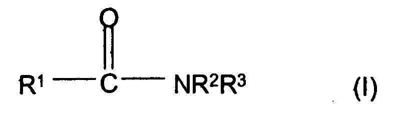 wobei
   R² für eine Gruppe der Formel CH₂=CR⁴- steht und R¹ und R³ unabhängig voneinander für H, Alkyl, Cycloalkyl, Heterocycloalkyl, Aryl oder Hetaryl stehen, oder R¹ und R³ gemeinsam mit der Amidgruppe, an die sie gebunden sind, für ein Lactam mit 5 bis 8 Ringatomen stehen,
b) wenigstens eine radikalisch polymerisierbare vernetzende Verbindung mit wenigstens zwei α,β-ethylenisch ungesättigten Doppelbindungen pro Molekül,
c) wenigstens eine Verbindung mit einer radikalisch polymerisierbaren α,β-ethylenisch ungesättigten Doppelbindung und mindestens einer kationogenen und/oder kationischen Gruppe pro Molekül,
in einem wässrigen Medium in Gegenwart wenigstens eines polymeren anionischen Dispergiermittels D). Sie eignen sich als Konditioniermittel für kosmetische Zubereitungen, insbesondere Shampoos.

Die WO 2005/058988 beschreibt ampholytische Copolymere, die einen molaren Über schuss an anionogenen und/oder anionischen Gruppen enthalten und die durch radikalische Polymerisation von
a) wenigstens einem verzweigten C₃-C₅-Alkylacrylat,
b) Acrylsäure und/oder Methacrylsäure
c) einer Monomerzusammensetzung, enthaltend
   c1) wenigstens eine Verbindung mit einer radikalisch polymerisierbaren, α,β-ethylenisch ungesättigten Doppelbindung und wenigstens einer anionogenen und/oder anionischen Gruppe pro Molekül und
   c2) wenigstens eine Verbindung mit einer radikalisch polymerisierbaren, α,β-ethylenisch ungesättigten Doppelbindung und wenigstens einer kationogenen und/oder kationischen Gruppe pro Molekül,
   erhältlich sind, wobei das molare Verhältnis von anionogenen und anionischen Gruppen der Komponente c1) zu kationogenen und kationischen Gruppen der Komponente c2) etwa 1 : 1-beträgt.

Die nicht vorveröffentlichten internationalen Anmeldungen WO 2007/010034 (PCT/EP2006/064504), WO 2007/010035 (PCT/EP2006/064506) und WO 2007/012610 (PCT/EP2006/064507) beschreiben anionisch ampholytische Copolymere, kationisch ampholytische Copolymere und deren Verwendung als Rheologiemodifizierungsmittel für haarkosmetische Zusammensetzungen.

Trotz der umfangreichen Bemühungen besteht nach wie vor Verbesserungsbedarf bei den aus dem Stand der Technik bekannten Polymeren zur Erzeugung elastischer Frisuren bei gleichzeitig starker Festigung (auch bei hoher Luftfeuchtigkeit). Für einen Erfolg versprechenden Einsatz in Haarsprayformulierungen sind zudem eine gute Treibgasverträglichkeit, eine gute Löslichkeit in Wasser oder wässrig/alkoholischen Lösungsmittelgemischen, die Eignung für einen Einsatz in low-VOC-Formulierungen und eine gute Auswaschbarkeit erwünscht. Gute Eigenschaften sind ebenso bezüglich der Konditionierung des Haars in seinen sensorisch erfassbaren Eigenschaften wie Griff, Volumen, Handhabbarkeit usw. gewünscht. Ferner sollen sich die Polymere durch eine gute Verträglichkeit mit anderen Formulierungsbestandteilen auszeichnen.

Überraschenderweise wurde gefunden, dass sich für die zuvor genannten Anforderungen besonders ampholytische Copolymere eignen, die einen molaren Überschuss an kationogenen/kationischen Gruppen gegenüber anionogenen/anionischen Gruppen aufweisen und die durch radikalische Polymerisation von
a) wenigstens einem α,β-ethylenisch ungesättigten Monomer der allgemeinen Formel I 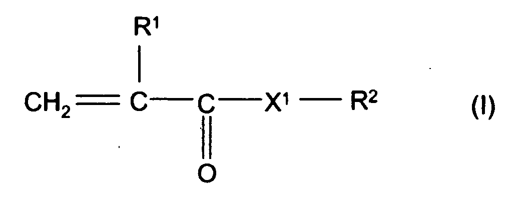 worin
   R¹ für Wasserstoff oder C₁-C₈-Alkyl steht,
   X¹ für O oder NR³ steht, wobei R³ für Wasserstoff, Alkyl, Cycloalkyl, Aryl oder Hetaryl steht,
   R² für verzweigtes C₃-C₅-Alkyl steht,
b) wenigstens einer Verbindung mit einer radikalisch polymerisierbaren, α,β-ethylenisch ungesättigten Doppelbindung und wenigstens einer kationogenen und/oder kationischen Gruppen pro Molekül, mit der Maßgabe, dass zumindest ein Teil der Verbindungen b) wenigstens ein quartäres Stickstoffatom aufweist,
c) wenigstens einer Verbindung mit einer radikalisch polymerisierbaren, α,β-ethylenisch ungesättigten Doppelbindung und wenigstens einer anionogenen und/oder anionischen Gruppe pro Molekül und
d) gegebenenfalls wenigstens einem amidgruppenhaltigen Monomer, das ausgewählt ist unter α,β-ethylenisch ungesättigten amidgruppenhaltigen Verbindungen der allgemeinen Formel II 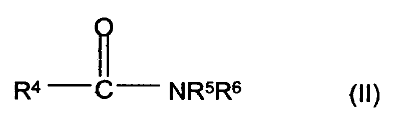 wobei
   einer der Reste R⁴ bis R⁶ für eine Gruppe der Formel CH₂=CR⁷- mit R⁷ = H oder C₁-C₄-Alkyl steht und die übrigen Reste R⁴ bis R⁶ unabhängig voneinander für H, Alkyl, Cycloalkyl, Heterocycloalkyl, Aryl oder Hetaryl stehen,
   wobei R⁴ und R⁵ gemeinsam mit der Amidgruppe, an die sie gebunden sind, auch für ein Lactam mit 5 bis 8 Ringatomen stehen können,
   wobei R⁵ und R⁶ gemeinsam mit dem Stickstoffatom, an das sie gebunden sind, auch für einen fünf bis siebengliedrigen Heterocyclus stehen können.
erhältlich sind.

In einer bevorzugten Ausführungsform der vorliegenden Erfindung enthält das ampholytische Copolymer wenigstens ein Monomer der Komponente d) einpolymerisiert.

Im Rahmen der vorliegenden Erfindung umfasst der Ausdruck Alkyl geradkettige und verzweigte Alkylgruppen. Geeignete kurzkettige Alkylgruppen sind z. B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, Isobutyl, sec.-Butyl, tert.-Butyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 2-Ethylpentyl, 1-Propylbutyl.

Verzweigtes C₃-C₅-Alkyl steht vorzugsweise für Isopropyl, Isobutyl, sec.-Butyl, tert.-Butyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl. Bevorzugt ist tert.-Butyl.

Geeignete längerkettige C₈-C₃₀-Alkylgruppen sind geradkettige und verzweigte Alkylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen. Dazu zählen z. B. n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignoceryl, Cerotinyl, Melissyl(en), etc.

Geeignete längerkettige C₈-C₃₀-Alkenylgruppen sind geradkettige und verzweigte Alkenylgruppen, die einfach, zweifach oder mehrfach ungesättigt sein können. Bevorzugt handelt es sich um überwiegend lineare Alkenylreste, wie sie auch in natürlichen oder synthetischen Fettsäuren und Fettalkoholen sowie Oxoalkoholen vorkommen. Dazu zählen insbesondere Octenyl, Nonenyl, Decenyl, Undecenyl, Dodecenyl, Tridecenyl, Tetradecenyl, Pentadecenyl, Hexadecenyl, Heptadecenyl, Octadecenyl, Nonadecenyl, n-Eicosenyl, n-Docosenyl, n-Tetracosenyl, Hexacosenyl, Triacontenyl, etc.

Cycloalkyl steht vorzugsweise für C₅-C₈-Cycloalkyl, wie Cyclopentyl, Cyclohexyl, Cycloheptyl oder Cyclooctyl.

Aryl umfasst unsubstituierte und substituierte Arylgruppen und steht vorzugsweise für Phenyl, Tolyl, Xylyl, Mesityl, Naphthyl, Fluorenyl, Anthracenyl, Phenanthrenyl, Naphthacenyl und insbesondere für Phenyl, Tolyl, Xylyl oder Mesityl.

Der Begriff "N,N-Dialkylamide" umfasst auch Verbindungen, bei denen der Amidstickstoff Teil eines fünf- bis siebengliedrigen Heterocyclus ist, der zusätzlich ein Heteroatom, ausgewählt unter Sauerstoff, Schwefel und NR^{a}, worin R^{a} für Wasserstoff, Alkyl oder Cycloalkyl steht, enthalten kann.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar. Die erfindungsgemäßen Copolymere A) sind im Allgemeinen wasserlöslich.

Die erfindungsgemäßen Copolymere weisen in einer speziellen Ausführung keine siliciumatomhaltigen Gruppen auf.

Das zur Herstellung der erfindungsgemäßen Copolymere eingesetzte Monomerengemisch weist Monomere mit kationogenen und/oder kationischen Gruppen und Monomere mit anionogenen und/oder anionischen Gruppen auf. Die Menge an zur Polymerisation eingesetzten Monomeren mit ionogenen und/oder ionischen Gruppen wird dabei so bemessen, dass, bezogen auf die insgesamt zur Polymerisation eingesetzten Monomere, der Molanteil an kationogenen und kationischen Gruppen größer ist als der Molanteil an anionogenen und anionischen Gruppen. Die erfindungsgemäßen Copolymere weisen daher im Mittel einen molaren Überschuss an kationogenen/kationischen Gruppen gegenüber anionogenen/anionischen Gruppen auf. Bevorzugt beträgt das molare Verhältnis von kationogenen/kationischen Gruppen zu anionogenen/anionischen Gruppen wenigstens 1,01 : 1, besonders bevorzugt wenigstens 1,2 : 1, insbesondere wenigstens 1,4 : 1, speziell wenigstens 1,5:1, spezieller wenigstens 2 : 1.

Überraschenderweise wurde gefunden, dass man Copolymere mit besonders vorteilhaften Eigenschaften erhält, wenn zur Polymerisation Monomere mit kationischen Gruppen eingesetzt werden, die wenigstens ein quartäres Stickstoffatom aufweisen. Bevorzugt handelt es sich bei den kationogenen und/oder kationischen Gruppen der Komponente b) um stickstoffhaltige Gruppen, wie primäre, sekundäre und tertiäre Aminogruppen sowie quartäre Ammoniumgruppen. Erfindungsgemäß weist zumindest ein Teil der Monomere b) quartäre Ammoniumgruppen auf. Quartäre Ammoniumgruppen, d. h. geladene kationische Gruppen, lassen sich aus den Aminstickstoffen durch Quaternisierung mit Alkylierungsmitteln erzeugen. Dazu zählen C₁-C₄-Alkylhalogenide oder -sulfate, wie Ethylchlorid, Ethylbromid, Methylchlorid, Methylbromid, Dimethylsulfat und Diethylsulfat. Ein bevorzugtes Quaternisierungsmittel ist Diethylsulfat. Geladene kationische Gruppen (jedoch keine quartären Ammoniumgruppen im Sinne der Erfindung) lassen sich aus den Aminstickstoffen auch durch Protonierung mit Säuren erzeugen. Geeignete Säuren sind z. B. Carbonsäuren, wie Milchsäure, oder Mineralsäuren, wie Phosphorsäure, Schwefelsäure und Salzsäure.

### Monomer a)

Die erfindungsgemäßen Copolymere enthalten wenigstens eine Verbindung einpolymerisiert, die vorzugsweise ausgewählt ist unter Isopropylacrylat, Isopropylmethacrylat, Isopropylacrylamid, Isopropylmethacrylamid, Isobutylacrylat, Isobutylmethacrylat, Isobutylacrylamid, Isobutylmethacrylamid, sek.-Butylacrylat, sek.-Butylmethacrylat, sek.-Butylacrylamid, sek.-Butylmethacrylamid, tert.-Butylacrylat, tert.-Butylmethacrylat, tert.-Butylacrylamid, tert.-Butylmethacrylamid, 1-Methylbutylacrylat, 1-Methylbutylmethacrylat, 1-Methylbutylacrylamid, 1-Methylbutylmethacrylamid, 2-Methylbutylacrylat, 2-Methylbutylmethacrylat, 2-Methylbutylacrylamid, 2-Methylbutylmethacrylamid, 3-Methylbutylacrylat, 3-Methylbutylmethacrylat, 3-Methylbutylacrylamid, 3-Methylbutylmethacrylamid, 1,1-Dimethylpropylacrylat, 1,1-Dimethylpropylmethacrylat, 1,1-Dimethylpropylacrylamid, 1,1-Dimethylpropylmethacrylamid, 2,2-Dimethylpropylacrylat, 2,2-Dimethylpropylmethacrylat, 2,2-Dimethylpropylacrylamid, 2,2-Dimethylpropylmethacrylamid, und Mischungen davon. Besonders bevorzugt ist tert.-Butylacrylat und sind Mischungen, die tert.-Butylacrylat enthalten.

Die erfindungsgemäßen Copolymere enthalten vorzugsweise 15 bis 90 Gew.-%, besonders bevorzugt 20 bis 85 Gew.-%, insbesondere 25 bis 75 Gew.-%, bezogen auf das Gesamtgewicht der zur Polymerisation eingesetzten Monomere, wenigstens eines Monomers a) einpolymerisiert.

### Monomer b)

Die erfindungsgemäßen Copolymere enthalten als Verbindung b) wenigstens eine Verbindung mit einer radikalisch polymerisierbaren, α,β-ethylenisch ungesättigten Doppelbindung und mindesten einer kationogenen und/oder kationischen Gruppe pro Molekül einpolymerisiert. Dabei weist erfindungsgemäß zumindest ein Teil der Verbindungen b) ein quartäres Stickstoffatom auf.

Die erfindungsgemäßen Copolymere enthalten vorzugsweise 3 bis 98 Gew.-%, besonders bevorzugt 5 bis 90 Gew.-%, insbesondere 7 bis 80 Gew.-%, bezogen auf das Gesamtgewicht der zur Polymerisation eingesetzten Monomere, wenigstens eines Monomers b) einpolymerisiert.

Vorzugsweise liegen bis 100 Gew.-%, besonders bevorzugt 5 bis 100 Gew.-%, beispielsweise 10 bis 99 Gew.-%, der Monomere b), bezogen auf das Gesamtgewicht der Monomere b), in quatemisierter Form vor.

Vorzugsweise ist die Komponente b) ausgewählt unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen, den Quaternisierungsprodukten dieser Monomere und Mischungen davon.

In einer bevorzugten Ausführungsform umfasst die Komponente b) als vinylsubstituierte heteroaromatische Verbindung wenigstens eine N-Vinylimidazol-Verbindung. In einer speziellen Ausführungsform ist die Komponente b) ausgewählt unter N-Vinylimidazol-Verbindungen und Mischungen, die wenigstens eine N-Vinylimidazol-Verbindung enthalten.

Geeignete N-Vinylimidazol-Verbindungen sind Verbindungen der Formel 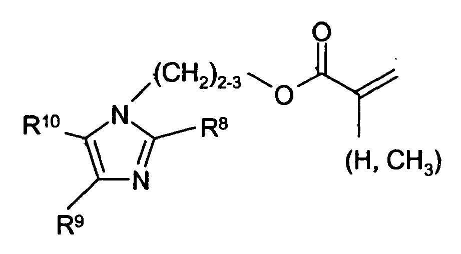 worin R⁸ bis R¹⁰ unabhängig voneinander für Wasserstoff, C₁-C₄-Alkyl oder Phenyl stehen. Bevorzugt stehen R⁸ bis R¹⁰ für Wasserstoff.

Des Weiteren bevorzugt enthält das Copolymer als Monomer b) wenigstens eine N-Vinylimidazol-Verbindung der allgemeinen Formel (III) 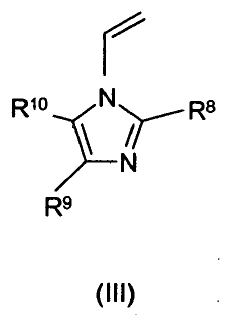 einpolymerisiert, worin R⁸ bis R¹⁰ unabhängig voneinander für Wasserstoff, C₁-C₄-Alkyl oder Phenyl stehen.

Beispiele für Verbindungen der allgemeinen Formel (III) sind folgender Tabelle 1 zu entnehmen:

**Tabelle 1**

| R⁸ | R⁹ | R¹⁰ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Bevorzugt als Monomer b) ist 1-Vinylimidazol (N-Vinylimidazol) und sind Mischungen, die N-Vinylimidazol enthalten.

Geeignete Monomere b) sind auch die durch Protonierung oder Quaternisierung der zuvor genannten N-Vinylimidazolverbindungen erhältlichen Verbindungen. Beispiele für solche geladenen Monomere b) sind quaternisierte Vinylimidazole, insbesondere 3-Methyl-1-vinylimidazoliumchlorid, -methosulfat und -ethosulfat. Geeignete Säuren und Alkylierungsmittel sind die zuvor aufgeführten.

Geeignete Verbindungen b) sind weiterhin die Ester von α,β-ethylenisch ungesättigten Mono- und Dicarbonsäuren mit Aminoalkoholen. Bevorzugte Aminoalkohole sind C₂-C₁₂-Aminoalkohole, welche am Aminstickstoff C₁-C₈-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden als Säurekomponente Acrylsäure, Methacrylsäure und deren Gemische eingesetzt.

Bevorzugte Monomere b) sind N-tert.-Butylaminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Besonders bevorzugt sind N-tert.-Butylaminoethyl(meth)acrylat und N,N-Dimethylaminoethyl(meth)acrylat. Bevorzugte Monomere b) sind insbesondere auch die Quaternisierungsprodukte der zuvor genannten Verbindungen.

Geeignete Monomere b) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen.

Bevorzugt als Monomere b) sind z. B. N-[tert.-Butylaminoethyl](meth)acrylamid, N-[2-Dimethylamino)ethyl]acrylamid, N-[2-(Dimethylamino)ethyl]methacrylamid, N-[3-(Dimethylamino)propyl]acrylamid, N-[3-(Dimethylamino)propyl]methacrylamid, N-[4-(Dimethylamino)butyl]acrylamid, N-[4-(Dimethylamino)butyl]methacrylamid, N-[2-(Diethylamino)ethyl]acrylamid, N-[4-(Dimethylamino)cyclohexyl]acrylamid und N-[4-(Dimethylamino)cyclohexyl]methacrylamid. Besonders bevorzugt sind N-[3-(Dimethylamino)propyl]acrylamid und N-[3-(Dimethylamino)propyl]methacrylamid (DMAPMAM).

Eine spezielle Ausführungsform betrifft Copolymere A), die N-[3-(Dimethylamino)propyl]acrylamid und N-[3-(Dimethylamino)propyl]methacrylamid einpolymerisiert enthalten. In einer ganz speziellen Ausführung besteht die Komponente b) nur aus N-[3-(Dimethylamino)propyl]acrylamid und/oder N-[3-(Dimethylamino)propyl]methacrylamid.

Geeignete Monomere b) sind weiterhin N,N-Diallylamine und N,N-Diallyl-N-alkylamine und deren Säureadditionssalze und Quaternisierungsprodukte. Alkyl steht dabei vorzugsweise für C₁-C₂₄-Alkyl. Bevorzugt sind N,N-Diallyl-N-methylamin und N,N-Diallyl-N,N-dimethylammonium-Verbindungen, wie z. B. die Chloride und Bromide. Besonders bevorzugt ist N,N-Diallyl-N-methylamin.

Geeignete Monomere b) sind weiterhin von Vinylimidazolen verschiedene vinyl- und allylsubstituierte Stickstoffheterocyclen, wie 2- und 4-Vinylpyridin, 2- und 4-Allylpyridin, und die Salze davon.

Bevorzugt umfasst die Komponente b) wenigstens ein Monomer, das ausgewählt ist unter N,N-Dimethylaminoethyl(meth)acrylat, N-[3-(Dimethylamino)propyl](meth)acrylamid, quaternisiertem N,N-Dimethylaminoethyl(meth)acrylat, quaternisiertem N-[3-(Dimethylamino)propyl](meth)acrylamid und Mischungen davon.

Des Weiteren bevorzugt umfasst die Komponente b) mit Methylchlorid, Dimethylsulfat oder Diethylsulfat quaternisiertes N,N-Dimethylaminoethyl(meth)acrylat. Hier und im Folgenden werden in Bezug auf den Begriff "mit Diethylsulfat quaternisiertes Dimethylaminoethylmethacrylat" die Begriffe "quat DMAEMA" und "Quat 311" synonym gebraucht. Speziell umfasst die Komponente b) mit Diethylsulfat quaternisiertes N,N-Dimethylaminoethyl(meth)acrylat.

Die erfindungsgemäßen Copolymere enthalten vorzugsweise 2 bis 97 Gew.-%, besonders bevorzugt 3 bis 96 Gew.-%, insbesondere 4 bis 60 Gew.-%, bezogen auf das Gesamtgewicht der zur Polymerisation eingesetzten Monomere, wenigstens eines Monomers b) einpolymerisiert.

### Monomer c)

Die erfindungsgemäßen Copolymere enthalten als Verbindung c) wenigstens eine Verbindung mit einer radikalisch polymerisierbaren, α,β-ethylenisch ungesättigten Doppelbindung und wenigstens einer anionogenen und/oder anionischen Gruppe pro Molekül. Die Komponente c) wird vorzugsweise in einer Menge 0,1 bis 30 Gew.-%, besonders bevorzugt 1 bis 25 Gew.-%, insbesondere 1,5 bis 20 Gew.-%, eingesetzt.

Vorzugsweise umfasst die Komponente c) wenigstens eine Verbindung, die ausgewählt ist unter monoethylenisch ungesättigten Carbonsäuren, Sulfonsäuren, Phosphonsäuren und Mischungen davon.

Zu den Monomeren c) zählen monoethylenisch ungesättigte Mono- und Dicarbonsäuren mit 3 bis 25, vorzugsweise 3 bis 6 C-Atomen, die auch in Form ihrer Salze oder Anhydride eingesetzt werden können. Beispiele hierfür sind Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure. Zu den Monomeren c) zählen weiterhin die Halbester von monoethylenisch ungesättigten Dicarbonsäuren mit 4 bis 10, vorzugsweise 4 bis 6 C-Atomen, z. B. von Maleinsäure wie Maleinsäuremonomethylester. Zu den Monomeren c) zählen auch monoethylenisch ungesättigte Sulfonsäuren und Phosphonsäuren, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Styrolsulfonsäure, 2-Acrylamido-2-methylpropansulfonsäure, Vinylphosphonsäure und Allylphosphonsäure. Zu den Monomeren c) zählen auch die Salze der zuvor genannten Säuren, insbesondere die Natrium-, Kalium- und Ammoniumsalze sowie die Salze mit Aminen. Die Monomere c) können als solche oder als Mischungen untereinander eingesetzt werden. Die angegebenen Gewichtsanteile beziehen sich sämtlich auf die Säureform.

Vorzugsweise umfasst die Komponente c) wenigstens eine Verbindung, die ausgewählt ist unter Acrylsäure, Methacrylsäure, Ethacrylsäure, α-Chloracrylsäure, Crotonsäure, Maleinsäure, Maleinsäureanhydrid, Fumarsäure, Itaconsäure, Citraconsäure, Mesaconsäure, Glutaconsäure, Aconitsäure, 2-Acrylamido-2-methylpropansulfonsäure, Vinylphosphonsäure und Mischungen davon.

Besonders umfasst die Komponente c) wenigstens eine Verbindung c), die ausgewählt ist unter Acrylsäure, Methacrylsäure und Mischungen davon. In einer speziellen Ausführung umfasst die Komponente c) Methacrylsäure oder besteht aus Methacrylsäure.

### Monomer d)

Die erfindungsgemäßen Copolymere enthalten vorzugsweise 5 bis 95 Gew.-%, besonders bevorzugt 10 bis 90 Gew.-%, bezogen auf das Gesamtgewicht der zur Polymerisation eingesetzten Verbindungen, wenigstens eines Monomers d) einpolymerisiert.

Bevorzugt sind die Verbindungen der Komponente d) ausgewählt unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

Bevorzugte Monomere d) sind N-Vinyllactame und deren Derivate, die z. B. einen oder mehrere Alkylsubstituenten, wie Methyl, Ethyl, n-Propyl, Isopropyl, n-Butyl, sek.-Butyl, tert.-Butyl etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Besonders bevorzugt werden N-Vinylpyrrolidon und/oder N-Vinylcaprolactam eingesetzt.

Geeignete Monomere d) sind weiterhin Acrylsäureamid und Methacrylsäureamid.

Geeignete N-Alkyl- und N,N-Dialkylamide α,β-ethylenisch ungesättigter Monocarbonsäuren, die zusätzlich zu dem Carbonyl-Kohlenstoffatom der Amidgruppe höchstens 7 weitere Kohlenstoffatome aufweisen, sind beispielsweise N-Methyl(meth)acrylamid, N-Ethyl(meth)acrylamid, N-Propyl(meth)acrylamid, N-(n-Butyl)(meth)acrylamid, N-tert-Butyl(meth)acrylamid, n-Pentyl(meth)acrylamid, n-Hexyl(meth)acrylamid, n-Heptyl(meth)acrylamid, N,N-Dimethyl(meth)acrylamid, N,N-Diethyl(meth)acrylamid, Piperidinyl(meth)acrylamid, Morpholinyl(meth)acrylamid und Mischungen davon.

Geeignete N-C₈-C₃₀-Alkyl- und N-(C₁-C₃₀)alkyl-N-(C₈-C₃₀)alkylamide c) sind z. B. n-Octyl(meth)acrylamid,1,1,3,3-Tetramethylbutyl(meth)acrylamid, 2-Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arachinyl(meth)acrylamid, Behenyl(meth)acrylamid, Lignocerenyl(meth)acrylamid, Cerotinyl(meth)acrylamid, Melissyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid, N-Methyl-N-(n-octyl)(meth)acrylamid, N,N-Di-(n-octyl)(meth)acrylamid und Mischungen davon.

Als Monomere d) geeignete offenkettige N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon. Bevorzugt wird N-Vinylformamid eingesetzt.

Als Monomere d) eignen sich auch Verbindungen der Formel

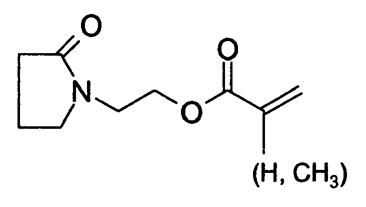

Besonders bevorzugt werden N-Vinylpyrrolidon, N-Vinylcaprolactam, N-Vinylformamid und die Verbindungen der obigen Formel eingesetzt.

### Monomer e)

Die erfindungsgemäßen Copolymere können zusätzlich wenigstens ein von den Komponenten a) bis d) verschiedenes, damit copolymerisierbares Monomer e) einpolymerisiert enthalten.

Vorzugsweise ist die Komponente e) ausgewählt unter von Komponente a) verschiedenen Estern α,β-ethylenisch ungesättigter Mono- und Dicarbonsäuren mit C₁-C₃₀-Alkanolen, ungesättigten C₈-C₃₀-Fettalkoholen und C₂-C₃₀-Alkandiolen, Amiden α,β-ethylenisch ungesättigter Mono- und Dicarbonsäuren mit C₂-C₃₀-Aminoalkoholen, die eine primäre oder sekundäre Aminogruppe aufweisen., Estern von Vinylalkohol und Allylalkohol mit C₁-C₃₀-Monocarbonsäuren, Vinylethern, Vinylaromaten, Vinylhalogeniden, Vinylidenhalogeniden, C₂-C₈-Monoolefinen, nicht aromatischen Kohlenwasserstoffen mit mindestens zwei konjugierten Doppelbindungen und Mischungen davon.

Geeignete zusätzliche Monomere e) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.

Geeignete zusätzliche Monomere e) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Geeignete Monomere e) sind auch Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20000 auf. Geeignete Polyetherole sind Polyalkylenglykole, wie Polyethylenglykole, Polypropylenglykole, Polytetrahydrofurane und Alkylenoxidcopolymere. Geeignete AIkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere.

Bevorzugt als Komponente e) sind Polyetheracrylate der allgemeinen Formel IV 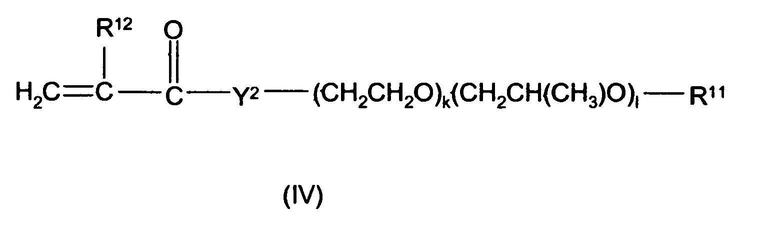 worin
die Reihenfolge der Alkylenoxideinheiten beliebig ist,
k und I unabhängig voneinander für eine ganze Zahl von 0 bis 1000 stehen, wobei die Summe aus k und I mindestens 5 beträgt,
R¹¹ für Wasserstoff, C₁-C₃₀-Alkyl oder C₅-C₈-Cycloalkyl steht,
R¹² für Wasserstoff oder C₁-C₈-Alkyl steht,
Y² für O oder NR¹³ steht, wobei R¹³ für Wasserstoff, C₁-C₃₀-Alkyl oder C₅-C₈-Cycloalkyl steht.

Bevorzugt steht k für eine ganze Zahl von 1 bis 500, insbesondere 3 bis 250. Bevorzugt steht I für eine ganze Zahl von 0 bis 100.

Bevorzugt steht R¹² für Wasserstoff, Methyl, Ethyl, n-Propyl, Isopropyl, n-Butyl, sek.-Butyl, tert.-Butyl, n-Pentyl oder n-Hexyl, insbesondere für Wasserstoff, Methyl oder Ethyl.

Vorzugsweise steht R¹¹ in der Formel IV für Wasserstoff, Methyl, Ethyl, n-Propyl, Isopropyl, n-Butyl, sec-Butyl, n-Pentyl, n-Hexyl, Octyl, 2-Ethylhexyl, Decyl, Lauryl, Palmityl oder Stearyl.

Vorzugsweise steht Y² in der Formel IV für O oder NH.

Geeignete Polyetheracrylate e) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R¹¹-OH hergestellt werden. Die Alkylenoxide können einzeln, alternierend nacheinander oder als Mischung eingesetzt werden. Die Polyetheracrylate e) können allein oder in Mischungen zur Herstellung der erfindungsgemäß eingesetzten Polymere verwendet werden.

Geeignete zusätzliche Monomere e) sind Methyl(meth)acrylat, Methylethacrylat, Ethyl(meth)acrylat, Ethylethacrylat, n-Butyl(meth)acrylat, tert.-Butylmethacrylat, tert.-Butylethacrylat, n-Octyl(meth)acrylat, 1,1,3,3-Tetramethylbutyl(meth)acrylat, 2-Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arachinyl(meth)acrylat, Behenyl(meth)acrylat, Lignocerenyl(meth)acrylat, Cerotinyl(meth)acrylat, Melissyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon. Bevorzugte Monomere e) sind die Ester α,β-ethylenisch ungesättigter Mono- und Dicarbonsäuren mit C₁-C₄-Alkanolen.

Geeignete zusätzliche Monomere e) sind weiterhin Vinylacetat, Vinylpropionat, Vinylbutyrat und Mischungen davon.

Geeignete zusätzliche Monomere e) sind weiterhin Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere e) können einzeln oder in Form von beliebigen Mischungen eingesetzt werden.

Bevorzugt enthalten die erfindungsgemäßen Copolymere wenigstens eine Verbindung e) einpolymerisiert, die ausgewählt ist unter C₁-C₃-Alkylmethacrylaten, Hydroxy-C₁-C₃-alkylmethacrylaten und Mischungen davon. Besonders bevorzugt sind Ethylmethacrylat, Hydroxyethylmethacrylat und Mischungen davon. Insbesondere wird Ethylmethacrylat eingesetzt. Die erfindungsgemäßen Copolymere enthalten diese Monomere vorzugsweise in einer Menge von 0 bis 50 Gew.-%, besonders bevorzugt 0 bis 45 Gew.-%, bezogen auf das Gesamtgewicht der zur Polymerisation eingesetzten Verbindungen, einpolymerisiert.

Die erfindungsgemäßen Copolymere enthalten vorzugsweise 0 bis 25 Gew.-%, besonders bevorzugt 0 bis 20 Gew.-%, insbesondere 0 bis 15 Gew.-%, bezogen auf das Gesamtgewicht der zur Polymerisation eingesetzten Monomere, wenigstens eines Monomers e) einpolymerisiert. Wenn ein Monomer e) eingesetzt wird, dann vorzugsweise in einer Menge von mindestens 0,1 Gew.-%, besonders bevorzugt mindestens 1 Gew.-% und insbesondere wenigstens 5 Gew.-%.

### Vernetzer f)

Die erfindungsgemäßen Copolymere können gewünschtenfalls wenigstens einen Vernetzer, d. h. eine Verbindung mit zwei oder mehr ais zwei ethylenisch ungesättigten, nichtkonjugierten Doppelbindungen einpolymerisiert enthalten.

Vorzugsweise werden Vernetzer in einer Menge von 0,01 bis 3 Gew.-%, besonders bevorzugt 0,01 bis 2 Gew.-%, insbesondere 0,1 bis 2 Gew.-% und speziell 0,1 bis 1 Gew.-%, bezogen auf das Gesamtgewicht der zur Polymerisation eingesetzten Monomere, verwendet.

Geeignete Vernetzer f) sind zum Beispiel Acrylester, Methacrylester, Allylether oder Vinylether von mindestens zweiwertigen Alkoholen. Die OH-Gruppen der zugrunde liegenden Alkohole können dabei ganz oder teilweise verethert oder verestert sein; die Vernetzer enthalten aber mindestens zwei ethylenisch ungesättigte Gruppen.

Beispiele für die zugrunde liegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, . 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thiapentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrunde liegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer f) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele Für solche Alkohole sind Allylalkohol, 1-Buten-3-ol, 5-Hexen-1-ol, 1-Octen-3-ol, 9-Decen-1-ol, Dicyclopentenylafkohol, 10-Undecen-1-ol, Zimtalkohol, Citronellol, Crotylalkohol odercis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer f) sind Ester ungesättigter Carbonsäuren mit den oben beschriebenen mehrwertigen Alkoholen, beispielsweise der Ölsäure, Crotonsäure, Zimtsäure oder 10-Undecensäure.

Weitere geeignete Vernetzer f) sind Urethandiacrylate und Urethanpolyacrylate, wie sie z. B. unter der Bezeichnung Laromer® kommerziell erhältlich sind.

Geeignet als Vernetzer f) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer f) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer f) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenhamstofi oder N,N'-Divinylpropylenhamstofi.

Weitere geeignete Vernetzer f) sind Divinyldioxan, Tetraallylsilan oder Tetravinylsilan.

Selbstverständlich können auch Mischungen der vorgenannten Verbindungen f) eingesetzt werden. Vorzugsweise werden wasserlösliche Vernetzer f) eingesetzt.

Besonders bevorzugt eingesetzte Vernetzer f) sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer f) sind Pentaerythrittriallylether, Methylenbisacrylamid, N,N'-Divinylethylenharnstoff, Triallylamin und Triallylmonoalkylammoniumsalze und Acrylsäureester von Glykol, Butandiol, Trimethylolpropan oder Glycerin oder Acrylsäureester von mit Ethylenoxid und/oder-Epichlorhydrin umgesetztem Glykol, Butandiol, Trimethylolpropan oder Glycerin.

Bevorzugt sind Copolymere, die
- 20 bis 94,5 Gew.-%, besonders bevorzugt 25 bis 85 Gew.-%, wenigstens einer Verbindung a),
- 5 bis 79,5 Gew.-%, besonders bevorzugt 10 bis 35 Gew.-%, insbesondere 13 bis 30 Gew.-%, wenigstens einer Verbindung b),
- 0,5 bis 25 Gew.-%, besonders bevorzugt 1 bis 20 Gew.-%, insbesondere 2 bis 15 Gew.-%, wenigstens einer Verbindung c),
- 0 bis 74,5 Gew.-%, besonders bevorzugt 1 bis 60 Gew.-%, insbesondere 5 bis 50 Gew.-%, wenigstens einer Verbindung d),
- 0 bis 25 Gew.-%, besonders bevorzugt 0,1 bis 20 Gew.-%, insbesondere 1 bis 15 Gew.-%, wenigstens einer Verbindung e),
- 0 bis 5 Gew.-%, besonders bevorzugt 0,01 bis 3 Gew.-%, insbesondere 0,1 bis 2 Gew.-%, wenigstens eines Vernetzers f),
einpolymerisiert enthalten.

Eine bevorzugte Ausführung sind Copolymere, die aus Wiederholungseinheiten von
- tert.-Butyl(meth)acrylat, - wenigstens einer Verbindung b), die ausgewählt ist unter
   N,N-Dimethylaminoethyl(meth)acrylat,
   N-[3-(Dimethylamino)propyl(meth)acrylamid,
   N-(tert.-Butyl)aminoethyl(meth)acrylat, N-Vinylimidazol und Mischungen davon, wobei zumindest ein Teil der Verbindungen b) quaternisiert ist,
- Acrylsäure und/oder Methacrylsäure,
- Vinylpyrrolidon und/oder Vinylcaprolactam,
bestehen.

Eine weitere bevorzugte Ausführung sind Copolymere, die aus Wiedefiolungseinheiten von
- tert.-Butylacrylat,
- N,N-Dimethylaminoethylmethacrylat oder
   N-(3-(Dimethylamino)propyl]methacrylamid,
- quaternisiertem N,N-Dimethylaminoethylmethacrylat oder
   quaternisiertem N-[3-(Dimethylamino)propyl]methacrylamid,
- Methacrylsäure,
- Vinylpyrrolidon,
bestehen.

In einer speziellen Ausführung werden zur Herstellung der zuvor genannten Copolymere teilweise oder vollständig quaternisierte Monomere b) eingesetzt, wobei als Quaternisierungsmittel Dimethylsulfat oder Diethylsulfat, insbesondere Diethylsulfat, eingesetzt wurde.

Die Herstellung der erfindungsgemäßen Copolymere erfolgt nach üblichen, dem Fachmann bekannten Verfahren, z. B. durch Lösungs-, Fällungs-, Suspensions- oder Emulsionspolymerisation. Geeignet ist auch die W/W-Polymerisation in Wasser mit einem geeigneten Verdrängungsmittel, z. B. einem Salz, wie NaCl.

Bevorzugte Lösemittel zur Lösungspolymerisation sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Diglycerin und Dioxan. Besonders bevorzugt ist die Polymerisation in Wasser oder in einem Alkohol oder einem Wasser/Alkohol-Gemisch, beispielsweise in einem Wasser/Ethanol-Gemisch. Die Polymerisationstemperaturen liegen bei der Lösungspolymerisation vorzugsweise in einem Bereich von etwa 30 bis 120 °C, besonders bevorzugt 40 bis 100 °C.

Die Fällungspolymerisation erfolgt vorzugsweise in einem weitgehend wasserfreien, aprotischen Lösungsmittel oder Lösungsmittelgemisch, vorzugsweise in Ethylacetat und/oder n-Butylacetat. Unter einem weitgehend wasserfreien, aprotischen Lösungsmittel oder Lösungsmittelgemisch wird ein Lösungsmittel oder Lösungsmittelgemisch mit einem Wassergehalt von höchstens 5 Gew.-% verstanden.

Bevorzugt erfolgt die Fällungspolymerisation bei einer Temperatur im Bereich von 70 bis 140 °C, bevorzugt 75 bis 100 °C, insbesondere von 80 bis 95 °C. Die resultierenden Polymerteilchen fallen aus der Reaktionslösung aus und können durch übliche Verfahren, wie Filtration mittels Unterdruck, isoliert werden. Zur Fällungspolymerisation können oberflächenaktive, polymere Verbindungen, vorzugsweise auf Polysiloxanbasis, eingesetzt werden. Bei der Fällungspolymerisation werden in der Regel Polymere mit höheren Molekulargewichten als bei der Lösungspolymerisation erhalten.

Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azobisisobutyronitril, 2,2'-Azo-bis-(2-amidinopropan)dihydrochlorid oder 2,2'-Azobis-(2-methylbutyronitril). Geeignet sind auch Initiatormischungen oder Redox-Initiator-Systeme, wie z. B. Ascorbinsäure/Eisen(II)-sulfat/Natriumperoxodisulfat, tert.-Butylhydroperoxid/Natriumdisulfit, tert.-Butylhydroperoxid/Natriumhydroxymethansulfinat, H₂O₂/Cu'.

Die Polymerisation kann prinzipiell bei dem durch die eingesetzten Monomere resultierenden pH-Wert erfolgen. Wird zur Polymerisation wenigstens ein N-Vinyllactam eingesetzt (= Komponente d)), so wird der pH-Wert des Polymerisationsmediums vorzugsweise auf einen Wert von 5 bis 8, bevorzugt 6 bis 7, eingestellt. Es ist vorteilhaft, den pH-Wert während der Polymerisation dann in diesem Bereich zu halten. Zur Einstellung des pH-Werts vor, während oder nach der Polymerisation eignen sich prinzipiell alle anorganischen oder organischen Basen (und gegebenenfalls Säuren), insbesondere solche, die außer einer etwaigen Salzbildung keine Reaktion mit den Monomeren eingehen. Geeignete Basen sind z. B. Alkali- und Erdalkalihydroxide, tertiäre Amine, wie Triethylamin, sowie Aminoalkohole, wie Triethanolamin, Methyldiethanolamin oder Dimethylethanolamin. Bevorzugt wird zur Einstellung des pH-Werts wenigstens ein tertiären Amin, das insbesondere ausgewählt ist unter N,N-Dimethylethanolamin, N-Methyldiethanolamin, Triethanolamin und Mischungen davon, eingesetzt.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

Der K-Wert der erfindungsgemäßen Copolymere ist 18 oder höher, bevorzugt 25 oder höher, besonders bevorzugt 42 oder höher. Der K-Wert der erfindungsgemäßen Copolymere ist 120 oder niedriger, bevorzugt 80 oder niedriger, besonders bevorzugt 70 oder niedriger. Am meisten bevorzugt sind K-Werte im Bereich von 42 bis 70 (,Bestim- , mung nach Fikentscher, Cellulosechemie, Bd. 13, S. 58 bis 64 (1932)). Die K-Wert-Bestimmung erfolgt dabei als 1%ige Lösung des Copolymers in N-Methylpyrrolidon.

Für wasserhaltige Mittel sind auch K-Werte im Bereich von 28 bis 42 bevorzugt.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden.

Wird bei der Herstellung der Polymere ein organisches Lösungsmittel eingesetzt, so kann dieses durch übliche, dem Fachmann bekannte Verfahren, z. B. durch Destillation bei vermindertem Druck, entfernt werden.

Die erhaltenen flüssigen Polymerzusammensetzungen können durch verschiedene Trocknungsverfahren, wie z. B. Sprühtrocknung, Fluidized Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall.

Ein weiterer Gegenstand der Erfindung ist ein kosmetisches oder pharmazeutisches Mittel, enthaltend
A) wenigstens ein ampholytisches Copolymer, wie zuvor definiert, und
B) wenigstens einen kosmetisch akzeptablen Träger.

Die erfindungsgemäßen Mittel weisen vorzugsweise einen kosmetisch oder pharmazeutisch akzeptablen Träger B) auf, der ausgewählt ist unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Die erfindungsgemäßen Mittel weisen z. B. eine Öl- bzw. Fettkomponenten B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Geeignete hydrophile Träger B) sind ausgewählt unter Wasser, ein-, zwei- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

In einer erfindungsgemäßen Ausführungsform enthalten die Mittel 20 Gew.-% oder mehr Wasser.

In einer weiteren erfindungsgemäßen Ausführungsform enthalten die Mittel weniger als 20 Gew.-%, bevorzugt weniger als 10 Gew.-%, besonders bevorzugt weniger als 5 Gew.-% Wasser.

Ein geeignete Treibgas B) ist Propan/Butan.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden Eigenschaften eignen sich die zuvor beschriebenen Copolymere und Polyelektrolytkomplexe insbesondere als Zusatzstoffe für Haar- und Hautkosmetika.

Vorzugsweise liegen die erfindungsgemäßen Mittel in Form eines Gels, Schaums, einer Mousse, eines Sprays, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes Copolymer A), wenigstens einen wie vorstehend definierten Träger B) und wenigstens einen davon verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionem, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettem, Collagen, Eiweißhydrolysaten, Lipiden, Antioxidantien, Entschäumem, Antistatika, Emollienzien und Weichmachern.

Übliche Verdickungsmittel in derartigen Formulierungen sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Bevorzugt werden nichtionische Verdicker eingesetzt.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind z. B. 2,4,6- Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen Zusammensetzungen können als Wirkstoff, z. B. als kosmetischen und/oder pharmazeutischen Wirkstoff wenigstens ein Polymer enthalten, das sich von den erfindungsgemäßen Copolymeren A) unterscheidet. Dazu zählen ganz allgemein anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wassedösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle Carboxypolysiloxane, Copolymere aus Vinylpyrrolidon, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der (Meth)acrylsäure, C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiter hin geeignet sind VinylpyrrolidoNEthylmethacrylat/Methacrylsäure-Copolymere wie sie von der Fa. Stepan unter den Bezeichnungen Stepanhold-Extra und - R1 vertrieben werden und die Carboset®-Marken der Fa. BF Goodrich.

Geeignete kationische Polymere sind z. B. kationische Polymere mit der Bezeichnung Polyquaternium nach INCl, z. B. Copolymere aus Vinylpyrrolidon/N-Vinylimidazoliumsalzen (Luviquat® FC, Luviquat® HM, Luviquat® MS, Luviset Clear®, Luviquat Supreme ®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquatemium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia. Geeignet sind auch kationische Polyurethane, z.B. die in der W02006/069742 beschriebenen.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Weiterhin sind die Polymere und Polyelektrolytkomplexe geeignet als Hilfsmittel in der Pharmazie, bevorzugt als oder in Beschichtungsmittel(n) oder Bindemittel(n) für feste Arzneiformen. Sie können auch in Cremes und als Tablettenüberzugsmittel und Tablettenbindemittel verwendet werden.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Kajalstifte, Eyelinern, Rouges, Puder und Augenbrauenstifte.

Außerdem können die ampholytischen Copolymere verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen ampholytischen Copolymere zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein ampholytischen Copolymere in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der ampholytischen Copolymere besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den ampholytischen Copolymere und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen ampholytischen Copolymere auch mit herkömmlichen Polymeren abmischen, falls spezielle Eigenschaften eingestellt werden sollen.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem ampholytischen Copolymer in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der wässrigen Phase kann ein erfindungsgemäßes ampholytisches Copolymer eingesetzt werden.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pfanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Neben den ampholytischen Copolymeren können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein ampholytisches Copolymer sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampoo-Formulierungen Verdicker, wie z. B. Kochsalz, PEG-55, Propyleneglykol-Oleat, PEG-120-Methylglucosedioleat und andere, sowie Konservierungsmittel, weitere Wirk- und Hilfsstoffe und Wasser enthalten.

Nach einer besonders bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein ampholytisches Copolymer in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Haarsprays, Schaumfestigers, Haarmousses, Haargels, Shampoos, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-)Spray, (Aerosol-)Schaum, Mousse, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Copolymere, die 0,01 bis 3 Gew.-% wenigstens einer Verbindung f) einpolymerisiert enthalten, sind besonders als Festiger und/oder Conditioner in Haarbehandlungsmitteln geeignet. Ebenso sind Copolymere, die einen K-Wert von 42 oder mehr haben, besonders als Festiger und/oder Conditioner in Haarbehandlungsmitteln geeignet. Diese Haarbehandlungsmittel sind ausgewählt aus Haargel, Shampoo, Schaumfestiger, Haarwasser, Haarspray, Haarschaum oder Mousse.

Die erfindungsgemäßen haarkosmetischen Formulierungen enthalten in einer bevorzugten Ausführungsform
a) 0,05 bis 20 Gew.-% wenigstens eines ampholytischen Copolymers, wie zuvor definiert,
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0 bis 3 Gew.-% wenigstens eines Verdickers, sowie
f) bis zu 25 Gew.-% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, farbstoffe, Viskositätsregulierer, Gelbildner, Farbstoffe, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen ampholytischen Copolymere und Polyelektrolytkomplexe eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Spray-Zubereitungen
a) 0,1 bis 10 Gew.-% wenigstens eines ampholytischen Copolymers, wie zuvor definiert,
b) 20 bis 99,9 Gew.-% Wasser und/oder Alkohol,
c) 0 bis 70 Gew.-% wenigstens eines Treibmittel,
d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält
a) 0,1 bis 10 Gew.-% wenigstens eines ampholytischen Copolymers, wie zuvor definiert,
b) 55 bis 94,8 Gew.-% Wasser und/oder Alkohol,
c) 5 bis 20 Gew.-% eines Treibmittel,
d) 0,1 bis 5 Gew.-% eines Emulgators,
e) 0 bis 10 Gew.-% weitere Bestandteile.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe,z. B. Cetheth-1, Polyethylenglycolcetylether; Cetearethe, z. B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quatemium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 10 Gew.-% wenigstens eines ampholytischen Copolymers, wie zuvor definiert,
b) 80 bis 99,9 Gew.-% Wasser und/oder Alkohol,
c) 0 bis 3 Gew.-%, bevorzugt 0,05 bis 2 Gew.-%, eines Gelbildners,
d) 0 bis 20 Gew.-% weitere Bestandteile.

Im Allgemeinen wirken die erfindungsgemäßen Copolymere bereits "selbstverdickend", so dass in vielen Fällen bei der Herstellung von Gelen auf den Einsatz von Gelbildnern verzichtet werden kann. Ihr Einsatz kann jedoch von Vorteil sein, um spezielle rheologische oder andere anwendungstechnische Eigenschaften der Gele einzustellen. Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44.

Copolymere, die 0,01 bis 3 Gew.-% wenigstens einer Verbindung f) einpolymerisiert enthalten, sind besonders als Rheologiemodifizierer in Hautreinigungsmitteln, Mitteln zur Pflege und zum Schutz der Haut, Nagelpflegemittel, Zubereitungen für die dekorative Kosmetik und Haarbehandlungsmitteln geeignet.

Die erfindungsgemäßen ampholytischen Copolymere können in kosmetischen Zubereitungen als Konditioniermittel eingesetzt werden.

Die erfindungsgemäßen ampholytischen Copolymere, wie zuvor definiert, können bevorzugt in Shampooformulierungen als Festigungs- und/oder Konditioniermittel eingesetzt werden. Bevorzugte Shampooformulierungen enthalten
a) 0,05 bis 10 Gew.-% wenigstens eines ampholytischen Copolymers, wie zuvor definiert,
b) 25 bis 94,95 Gew.-% Wasser,
c) 5 bis 50 Gew.-% Tenside,
d) 0 bis 5 Gew.-% eines weiteren Konditioniermittels,
e) 0 bis 10 Gew.-% weitere kosmetische Bestandteile.

In den Shampooformulierungen können alle in Shampoos üblicherweise eingesetzten anionischen, neutralen, amphoteren oder kationischen Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den ampholytischen Copolymeren eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquatemium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Ein weiterer Gegenstand der Erfindung ist die Verwendung eines ampholytischen Copolymers, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

Die Erfindung wird anhand der folgenden nicht einschränkenden Beispiele näher erläutert.

### Beispiele

### Allgemeine Herstellungsvorschrift:

### Beispiel 6: (TBA/VP/DMAPMAM/quat DMAEMA/MAS = 47 : 25 : 5 : 20 : 3)

| | | |
|---|---|---|
| Vorlage | 73 g | Wasser |
| | 112 g | Ethanol |
| | 23 g | Zulauf 1 |
| | 26 g | Zulauf 2 |
| | | |
| Zulauf 1 | 225 g | tert.-Butylacrylat |
| | 120 g | Vinylpyrrolidon |
| | 24 g | N,N-Dimethylaminopropylmethacrylamid |
| | 174 g | 50%ige Quat 311-Lösung |
| | 14,4 g | Methacrylsäure |
| | 100 g | Ethanol |
| | | |
| Zulauf 2 | 150 g | Wasser |
| | 300 g | Ethanol |
| | 0,96 g | tert.-Butylperpivalat 75%ig |
| | | |
| Zulauf 3 | 150 g | Wasser |
| | 300 g | Ethanol |
| | 2 g | tert.-Butylperpivalat 75%ig |
| | | |
| Zulauf 4 | 480 g | Ethanol |

In einer Rührapparatur mit Rückflusskühler, Innenthermometer und vier separaten Zulaufvorrichtungen wurde die Vorlage unter Rühren auf ca. 70 °C aufgeheizt. Nach dem Anpolymerisieren, erkennbar an einer leichten Viskositätserhöhung, wurde bei 70 °C der Rest von Zulauf 1 innerhalb von drei Stunden und der Rest von Zulauf 2 innerhalb von vier Stunden zugegeben. Die Reaktionslösung wurde noch ca. zwei Stunden bei 70 °C nachgerührt. Der Zulauf 3 wurde bei ca. 80 °C in 15 Minuten zudosiert und die Polymermischung noch ca. vier Stunden bei 80 °C gerührt. Nach Abkühlen auf ca. 60 °C wurde mit Phosphorsäure der pH-Wert auf 5,9 eingestellt. Anschließend wurde bei einer Außentemperatur von 120 °C Ethanol aus der Reaktionslösung durch Wasserdampfdestillation entfernt. Die Polymerlösung wurde auf ca. 40 °C abgekühlt, mit Ethanol (Zulauf 4) verdünnt und mit Wasser auf einen Feststoffgehalt von 30 % eingestellt. Analog wurden die Polymere der folgenden Tabelle 1 hergestellt.

**Tabelle 1**

| Bsp.- | TBA | VP Nr. | DMAPMAM | DMAEMA | quat DMAEMA | MAS | Neutralisierungsm./ Ng. oder pH | K-wert |
|---|---|---|---|---|---|---|---|---|
| 1 | 55 | 27 | | 10 | 5 | 3 | H₃PO₄ 60% | 19 |
| 2 | 50 | 25 | 18 | | 5 | 2 | H₃PO₄ pH 5,9 | 34,1 |
| 3 | 50 | 25 | 20 | | 3 | 2 | H₃PO₄ pH 6,3 | 34,4 |
| 4 | 50 | 27 | | 15 | 5 | 3 | Milchs. 90% | 25,1 |
| 5 | 47 | 25 | 10 | | 15 | 3 | H₃PO₄ pH 6,1 | 45 |
| 6 | 47 | 25 | 5 | | 20 | 3 | H₃PO₄ pH 5,9 | 41,8 |
| 7 | 45 | 30 | 15 | | 3 | 7 | Milchs. 90% | 33,9 |
| 8 | 45 | 30 | | 17 | 5 | 3 | H₃PO₄ 50% | 39,8 |
| 9 | 45 | 30 | 18 | | 4 | 3 | H₃PO₄ 50% | 43,7 |
| 10 | 45 | 30 | | 18 | 5 | 2 | H₃PO₄ 50% | 44,4 |
| 11 | 45 | 30 | 18 | | 5 | 2 | H₃PO₄ 50% | 36,7 |
| 12 | 45 | 25 | 15 | | 10 | 5 | H₃PO₄ pH 5,9 | 41,6 |
| 13 | 42 | 30 | 10 | | 15 | 3 | H₃PO₄ pH 5,9 | 39,9 |
| 14 | 42 | 30 | 5 | | 20 | 3 | H₃PO₄ pH 5,9 | 38,6 |
| 15 | 42 | 35 | 4 | | 17 | 2 | H₃PO₄ pH 5,7 | 39,8 |
| 16 | 42 | 30 | | | 25 | 3 | H₃PO₄ pH 5,0 | 37,5 |
| 17 | 37 | 35 | 10 | | 15 | 3 | H₃PO₄ pH 5,9 | 39,8 |
| 18 | 37 | 35 | 5 | | 20 | 3 | H₃PO₄ pH 6,0 | 40,6 |
| 19 | 37 | 40 | 4 | | 17 | 2 | H₃PO₄ pH 6,1 | 41,5 |
| 20 | 37 | 35 | | | 25 | 3 | H₃PO₄ pH 5,4 | 37,3 |

| | | | | | | | | |
|---|---|---|---|---|---|---|---|---|
| TBA tert.-Butylacrylat VP Vinylpyrrolidon DMAPMAM Dimethylaminopropylmethacrylamid DMAEMA Dimethylaminoethylmethacrylat quat. DMAEMA Dimethylaminoethylmethacrylat quaternisiert mit Diethylsulfat MAS Methacrylsäure Milchs. Milchsäure Ng. Neutralisierungsgrad in % K-Wert 1 %ig in N-Methylpyrrolidon | | | | | | | | |

### Beispiel 21: TBA/VP/DMAPMAM/Q311/MAS/ EGDMA = 35/37/10/15/3/ 0,1ppH

| | | |
|---|---|---|
| Vorlage | 100 g | Wasser |
| | 50 g | iso-Propanol |
| | 45 g | Zulauf 1 |
| | 2,5 g | Zulauf 2 |
| | | |
| Zulauf 1 | 164.6 g | Tert.-Butylacrylat |
| | 174 g | Vinylpyrrolidon |
| | 47g | N,N-Dimethylaminopropylmethacrylamid |
| | 140,8 g | 50%ige Quat 311-Lösung |
| | 14.4 g | Methacrylsäure |
| | 0,5 g | Ethylenglykoldimethacrylat |
| | 250 g | iso-Propanol |
| | 130 g | Wasser |
| | | |
| Zulauf 2 | 50 g | iso-Propanol |
| | 0,8 g | tert.- Butylperpivalat 75% ig |
| | | |
| Zulauf 3 | 50 g | Wasser |
| | 70 g | iso-Propanol |
| | 0,6g | tert.- Butylperpivalat 75% ig |

In einer Rührapparatur mit Rückflusskühler, Innenthermometer und drei separaten Zulaufvorrichtungen wurde die Vorlage unter Rühren auf ca. 70 °C aufgeheizt. Nach dem Anpolymerisieren, erkennbar an einer leichten Viskositätserhöhung, wurde bei 70 °C der Rest von Zulauf 1 innerhalb von drei Stunden und der Rest von Zulauf 2 innerhalb von vier Stunden zugegeben. Die Reaktionslösung wurde noch ca. zwei Stunden bei 70 °C nachgerührt. Der Zulauf 3 wurde bei ca. 80 °C in 15 Minuten zudosiert und die Polymermischung noch ca. vier Stunden bei 80 °C gerührt. Nach Abkühlen auf ca. 60 °C wurde mit Phosphorsäure der pH-Wert auf 5,9 eingestellt. Anschließend wurde bei einer Außentemperatur von 120 °C iso-Propanol aus der Reaktionslösung durch Wasserdampfdestillation entfernt. Die Polymerlösung wurde auf ca. 40 °C abgekühlt und mit Wasser auf einen Feststoffgehalt von 20 % eingestellt. Analog wurden die Polymere der folgenden Tabelle 2 hergestellt.

**Tabelle 2**

| Bsp.- Nr. | TBA | VP | DMAPMAM | quat DMAEMA | MAS | Vemetzer | Neutralisierungsm./ pH | K-Wert |
|---|---|---|---|---|---|---|---|---|
| 21 | 35 | 37 | 10 | 15 | 3 | EGDMA 0,1 ppH | H₃PO₄ pH 5,8 | 48,7 |
| 22 | 30 | 45 | 10 | 13 | 2 | EGDMA 0,2 ppH | H₃PO₄ pH 5,5 | 53,3 |
| 23 | 42 | 30 | 10 | 15 | 3 | PETAE 0,1 ppH | H₃PO₄ pH 5,8 | 47,6 |
| 24 | 35 | 37 | 10 | 15 | 3 | PETAE 0,1 ppH | H₃PO₄ pH 5,6 | 40,1 |

| | | | | | | | | |
|---|---|---|---|---|---|---|---|---|
| TBA tert.-Butylacrylat VP Vinylpyrrolidon DMAPMAM Dimethylaminopropylmethacrylamid quat. DMAEMA Dimethylaminoethylmethacrylat quaternisiert mit Diethylsulfat MAS Methacrylsäure K-Wert 1 %ig in N-Methylpyrrolidon EGDMA: Ethylenglykoldimethacrylat PETAE: Petaerythrittriallylether ppH Teile pro 100 Teile Monomer | | | | | | | | |

### Anwendungstechnische Beispiele:

Soweit nichts Gegenteiliges angegeben ist, sind Teile Gewichtsteile.

### A) Copolymere ohne Verbindung f)

### I) Verwendung in der Haarkosmetik:

### 1) VOC 80 Aerosol-Haarspray (Beispiele Nr. 1-20)

| | |
|---|---|
| Polymer 1, 2, 3, 4, 5, 6, 7, 8, 9, 10, 11, 12, 13,14, 15, 16, 17, 18, 19, 20 (30%ige wässrig-ethanol. Lösung) | 10,0 |
| Wasser | 13,0 |
| Dimethylether | 40,0 |
| Ethanol | 37,0 |

Weitere Zusatzstoffe:
Konservierungsmittel, lösliches ethoxiliertes Silikon, Parfüm, Entschäumer...

### 2) VOC 80 Aerosol-Haarspray (Beispiele Nr. 21 - 40) 3 % erfindungsgemäßes Polymer + 1 % Luviskol® VA 64

| | |
|---|---|
| Polymer 1, 2, 3, 4, 5, 6, 7, 8, 9, 10, 11, 12, 13,14, 15, 16, 17, 18, 19, 20 (30%ige wässrig-ethanol. Lösung) | 10,0 (3,0 Polymer + 7,0 Ethanol) |
| Luviskol® VA 64 (Pulver) | 1,0 |
| Wasser | 12,0 |
| Dimethylether | 40,0 |
| Ethanol | 37,0 |

Weitere Zusatzstoffe:
Konservierungsmittel, lösliches ethoxiliertes Silikon, Parfüm, Entschäumer...

### 3) VOC 55 Aerosol-Haarspray (Beispiele Nr. 41 - 60)

| | |
|---|---|
| Polymer 1, 2, 3, 4, 5, 6, 7, 8, 9, 10, 11, | |
| 12, 13, 14, 15, 16, 17, 18, 19, 20 (30%ige wässrig-ethanol. Lösung) | 8,75 |
| Wasser | 38,75 |
| Dimethylether | 40,0 |
| Ethanol | 12,5 |

Weitere Zusatzstoffe:
Konservierungsmittel, lösliches ethoxiliertes Silikon, Parfüm, Entschäumer...

### 4) VOC 55 Aerosol-Haarspray (Beispiele Nr. 61 - 80) 3 % erfindungsgemäßes Polymer + 1 % Luviskol® VA 64

| | |
|---|---|
| Polymer 1, 2, 3, 4, 5, 6, 7, 8, 9, 10, 11, 12, 13,14, 15, 16, 17, 18, 19, 20 (30%ige wässrig-ethanol. Lösung) | 10,0 |
| Luviskol® Plus | 1,0 |
| Wasser | 37,0 |
| Dimethylether | 40,0 |
| Ethanol | 12,0 |

Weitere Zusatzstoffe:
Konservierungsmittel, lösliches ethoxiliertes Silikon, Parfüm, Entschäumer...

### 5) VOC 55 Pumpspray (Beispiele Nr. 81 - 100)

| | |
|---|---|
| Polymer 1, 2, 3, 4, 5, 6, 7, 8, 9, 10, 11, 12, 13,14, 15, 16, 17, 18, 19, 20 (30%ige wässrig-ethanol. Lösung) | 10,0 |
| Wasser | 37,0 |
| Ethanol | 53,0 |

Weitere Zusatzstoffe:
Konservierungsmittel, lösliches ethoxiliertes Silikon, Parfüm, Entschäumer...

### 6) Schaumfestiger (Beispiele Nr. 101 - 120)

| | |
|---|---|
| Polymer 1, 2, 3, 4, 5, 6, 7, 8, 9, 10, 11, 12, 13,14, 15, 16, 17, 18, 19, 20 (30%ige wässrig-ethanol. Lösung) | 6,6 |
| Cremophor ® A 25 | 0,25 (Ceteareth 25, Fa. BASF) |
| Comperlan ® KD | 0,15 (Coamide DEA, Fa. Henkel) |
| Wasser | 80,0 |
| Dimethylether | 10,0 |

Weitere Zusatzstoffe: Parfüm, Konservierungsmittel...

Herstellung: Einwiegen und unter Rühren lösen. Abfüllen und Treibgas zusetzen.

### 7) Shampoo (Beispiele 121 - 140)

| | CTFA | Gew.-% |
|---|---|---|
| Phase 1: | | |
| Polymer aus Beispiel Nr. 1, 2, 3, 4, 5, 6, 7, 8, 9, 10, 11,12, 13,14, 15, 16, 17, 18, 19, 20 | | 1,0 |
| Wasser mit Triethanolamin (50%ig) auf pH 6,5 einstellen | | 48,0 |

| Phase 2: | | |
|---|---|---|
| Texapon® NSO 28%ig | Sodium Laureth Sulfphate/Henkel | 50,0 |
| Coperlan® KD Weiterer Zusatz: Parfüm, Konservierungsmittel, usw. | Coamide DEA/Henkel | 1,0 |

Herstellung:
Einwiegen und unter Rühren Phasen 1 und 2 getrennt lösen und mischen. Phase 2 langsam in Phase 1 einrühren.

### 8) Wasserfreies Haarspray (Beispiele Nr. 141 -160)

| | |
|---|---|
| Polymer 1, 2, 3, 4, 5, 6, 7, 8, 9, 10, 11, 12, 13,14, 15, 16, 17, 18, 19, 20 (getrocknetes Produkt, 100%) | 3,0 |
| Propan/Butan | 40,0 |
| Ethanol | 57,0 |
| Weitere Zusatzstoffe: | |

Konservierungsmittel, lösliches ethoxiliertes Silikon, Parfüm, Entschäumer...

### 9) Wasserfreies Haarspray (Beispiele Nr. 161 - 180) 2 % erfindungsgemäßes Polymer + 1 % Luviskol® Plus

| | |
|---|---|
| Polymer 1, 2, 3, 4, 5, 6, 7, 8, 9, 10, 11, 12, 13,14, 15, 16, 17, 18, 19, 20 (getrocknetes Produkt, 100%) | 2,0 |
| Luviskol® Plus | 1,0 |
| Propan/Butan | 40,0 |
| Ethanol | 57,0 |

Weitere Zusatzstoffe:
Konservierungsmittel, lösliches ethoxiliertes Silikon, Parfüm, Entschäumer...

### II) Verwendung in der Hautkosmetik:

### 10) Standard O/W-Creme (Beispiele Nr. 181 - 200)

| Öl-Phase: | | |
|---|---|---|
| | % | CTFA Name |
| Cremophor® A6 | 3,5 | Ceteareth-6 (and) Stearyl Alkohol |
| Cremophor® A25 | 3,5 | Ceteareth-25 |
| Glycerinmonostearat | 2,5 | Glyceryl Stearate |
| Paraffinöl | 7,5 | Paraffin Oil |
| Cetylalkohol | 2,5 | Cetyl Alkohol |
| Luvitol® EHO | 3,2 | Cetearyl Octanoate |
| Vitamin-E-acetate | 1,0 | Tocopheryl Acetate |
| Nip-Nip | 0,1 | Methyl- und Propyl-4-hydroxybenzoate (7:3) |

| Wasser-Phase: | | |
|---|---|---|
| | % | CTFA Name |
| Polymer 1, 2, 3, 4, 5, 6, 7, 8, 9, 10, 11, 12, 13, 14, 15, 16, 17, 18, 19, 20 (30%ige wässrig-ethanol. Lösung) | 3,0 | |
| Wasser | 74,6 | |
| 1,2-Propylenglykol | 1,5 | Propylene Glycol |
| Germall II | 0,1 | Imidazolidinyl-Urea |

Herstellung: Die Komponenten werden eingewogen und die Öl-Phase und Wasser-Phase getrennt bei einer Temperatur von ca. 80 °C unter Rühren homogenisieren. Die Wasser-Phase wird langsam in die Öl-Phase eingerührt und die Mischung unter Rühren auf Raumtemperatur abgekühlt.

### 11) Tageslotion (Beispiele Nr. 201 - 220)

| Öl-Phase: | % | CTFA Name |
|---|---|---|
| Cremophor® A6 | 1,5 | Ceteareth-6 (and) Stearyl Alkohol |
| Cremophor®A25 | 1,5 | Ceteareth-25 |
| Glycerinmonostearat | 5,0 | Glyceryl Stearate |
| Uvinul® MS 40 | 0,5 | Bezophenone-4 |
| Paraffinöl | 3,5 | Paraffin Oil |
| Cetylalkohol | 0,5 | Cetyl Alkohol |
| Luvitol® EHO | 10,0 | Cetearyl Octanoate |
| D-Panthenol 50 P | 3,0 | Panthenol und Propylenglykol |
| Vitamin-E-acetate | 1,0 | Tocopheryl Acetate |
| Tegiloxan 100 | 0,3 | Dimethicon |
| Nip-Nip | 0,1 | Methyl- und Propyl-4-hydroxybenzoate (7:3) |

| Wasser-Phase: | % | CTFA Name |
|---|---|---|
| Polymer 1, 2, 3, 4, 5, 6, 7, 8, 9, 10, 11, 12, 13,14, 15, 16, 17, 18, 19, 20 (30%ige wässrig-ethanol. Lösung) | 1,5 | |
| Wasser | 70,0 | |
| 1,2-Propylenglykol | 1,5 | Propylene Glycol |
| Germall II | 0,1 | Imidazolidinyl-Urea |

Herstellung: Die Komponenten werden eingewogen und die Öl-Phase und Wasser-Phase getrennt bei einer Temperatur von ca. 80°C unter Rühren homogenisieren. Die Wasser-Phase wird langsam in die Öl-Phase eingerührt und die Mischung unter Rühren auf Raumtemperatur abgekühlt.

### B) Copolymere mit Verbindung f)

### I) Verwendung in der Haarkosmetik:

### 12) Schaumfestiger (Beispiele Nr. 221 - 224)

| | |
|---|---|
| Polymer 21, 22, 23, 24 (20%ige wässrige Lösung) | 10,0 |
| Cremophor® A 25 | 0,25 (Ceteareth 25, Fa. BASF) |
| Comperlan® KD | 0,15 (Coamide DEA, Fa. Henkel) |
| Wasser | 79,6 |
| Dimethylether | 10,0 |

Weitere Zusatzstoffe: Parfüm, Konservierungsmittel...

Herstellung: Einwiegen und unter Rühren lösen. Abfüllen und Treibgas zusetzen.

### 13) Flüssiges Haargel (Beispiele Nr. 225 - 228)

| | |
|---|---|
| Natrosol ® 250 HR | 4,0 (Hydroxyethylcellulose) |
| Wasser | 64,0 |
| Polymer 21, 22, 23, 24 (20%ige wässrige Lösung) | 10,0 |
| Wasser | 20,0 |
| Glycerin | 2,0 |

Weitere Zusatzstoffe: Parfüm, Konservierungsmittel...

Herstellung: Natrosol in Wasser einwiegen und unter Rühren lösen. Polymer/Glycerin-Lösung wird separat hergestellt, dann langsam in Natrosol-Lösung einrühren lassen.

### II) Verwendung in der Hautkosmetik:

### 14) Standard O/W-Creme (Beispiele Nr. 229 - 232)

| Öl-Phase : | % | CTFA Name |
|---|---|---|
| Cremophor® A6 | 3,5 | Ceteareth-6 (and) Stearyl Alkohol |
| Cremophor® A25 | 3,5 | Ceteareth-25 |
| Glycerinmonostearat | 2,5 | Glyceryl Stearate |
| Paraffinöl | 7,5 | Paraffin Oil |
| Cetylalkohol | 2,5 | Cetyl Alkohol |
| Luvitol® EHO | 3,2 | Cetearyl Octanoate |
| Vitamin-E-acetate | 1,0 | Tocopheryl Acetate |
| Nip-Nip | 0,1 | Methyl- und Propyl-4-hydroxybenzoate (7:3) |

| Wasser-Phase: | % | CTFA Name |
|---|---|---|
| Polymer 21, 22, 23, 24 (20%ige wässrige Lösung) | 3,0 | |
| Wasser | 74,6 | |
| 1,2-Propylenglykol | 1,5 | Propylene Glycol |
| Germall II | 0,1 | Imidazolidinyl-Urea |

Herstellung: Die Komponenten werden eingewogen und die Öl-Phase und Wasser-Phase getrennt bei einer Temperatur von ca. 80 °C unter Rühren homogenisieren. Die Wasser-Phase wird langsam in die Öl-Phase eingerührt und die Mischung unter Rühren auf Raumtemperatur abgekühlt.

### 15) Tageslotion (Beispiele Nr. 233 - 236)

| Öl-Phase : | % | CTFA Name |
|---|---|---|
| Cremophor A6 | 1,5 | Ceteareth-6 (and) Stearyl Alkohol |
| Cremophor A25 | 1,5 | Ceteareth-25 |
| Glycerinmonostearat | 5,0 | Glyceryl Stearate |
| Uvinul® MS 40 | 0,5 | Bezophenone-4 |
| Paraffinöl | 3,5 | Paraffin Oil |
| Cetylalkohol | 0,5 | Cetyl Alkohol |
| Luvitol® EHO | 10,0 | Cetearyl Octanoate |
| D-Panthenol 50 P | 3,0 | Panthenol und Propyleneglykol |
| Vitamin-E-acetate | 1,0 | Tocopheryl Acetate |
| Tegiloxan 100 | 0,3 | Dimethicon |
| Nip-Nip | 0,1 | Methyl- und Propyl-4-hydroxybenzoate (7:3) |

| Wasser-Phase: | % | CTFA Name |
|---|---|---|
| Polymer 21, 22, 23, 24 (20%ige wässrige Lösung) | 1,5 | |
| Wasser | 70,0 | |
| 1,2-Propylenglykol | 1,5 | Propylene Glycol |
| Germall II | 0,1 | Imidazolidinyl-Urea |

Herstellung: Die Komponenten werden eingewogen und die Öl-Phase und Wasser-Phase getrennt bei einer Temperatur von ca. 80 °C unter Rühren homogenisieren. Die Wasser-Phase wird langsam in die Öl-Phase eingerührt und die Mischung unter Rühren auf Raumtemperatur abgekühlt.

## Patentansprüche

1. Ampholytisches Copolymer A, das einen molaren Überschuss an kationogenen/- kationischen Gruppen gegenüber anionogenen/anionischen Gruppen aufweist und das erhältlich ist durch radikalische Polymerisation von
a) wenigstens einem α,β-ethylenisch ungesättigten Monomer der allgemeinen Formel I 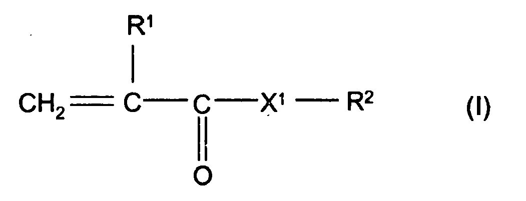 worin
R¹ für Wasserstoff oder C₁-C₈-Alkyl steht,
X¹ für O oder NR³ steht, wobei R³ für Wasserstoff, Alkyl, Cycloalkyl, Aryl oder Hetaryl steht,
R² für verzweigtes C₃-C₅-Alkyl steht,
b) wenigstens einer Verbindung mit einer radikalisch polymerisierbaren, α,β-ethylenisch ungesättigten Doppelbindung und wenigstens einer kationogenen und/oder kationischen Gruppen pro Molekül, mit der Maßgabe, dass zumindest ein Teil der Verbindungen b) wenigstens ein quartäres Stickstoffatom aufweist,
c) wenigstens einer Verbindung mit einer radikalisch polymerisierbaren, α,β-ethylenisch ungesättigten Doppelbindung und wenigstens einer anionogenen und/oder anionischen Gruppe pro Molekül und
d) gegebenenfalls wenigstens einem amidgruppenhaltigen Monomer, das ausgewählt ist unter α,β-ethylenisch ungesättigten amidgruppenhaltigen Verbindungen der allgemeinen Formel II 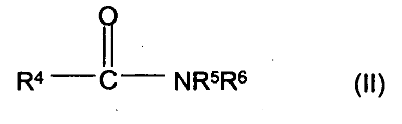 wobei
einer der Reste R⁴ bis R⁶ für eine Gruppe der Formel CH₂=CR⁷- mit R⁷ = H oder C₁-C₄-Alkyl steht und die übrigen Reste R⁴ bis R⁶ unabhängig voneinander für H, Alkyl, Cycloalkyl, Heterocycloalkyl, Aryl oder Hetaryl stehen,
wobei R⁴ und R⁵ gemeinsam mit der Amidgruppe, an die sie gebunden sind, auch für ein Lactam mit 5 bis 8 Ringatomen stehen können,
wobei R⁵ und R⁶ gemeinsam mit dem Stickstoffatom, an das sie gebunden sind, auch für einen fünf- bis siebengliedrigen Heterocyclus stehen können.

2. Copolymer nach Anspruch 1, wobei die Komponente a) wenigstens ein Monomer der Formel I umfasst, worin R² für tert.-Butyl steht.

3. Copolymer nach einem der vorhergehenden Ansprüche, wobei die Komponente a) tert.-Butylacrylat umfasst oder aus tert.-Butylacrylat besteht.

4. Copolymer nach einem der vorhergehenden Ansprüche, wobei die Komponente b) ausgewählt ist unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen, den Quatemisierungsprodukten dieser Monomere und Mischungen davon.

5. Copolymer nach einem der vorhergehenden Ansprüche, wobei die Komponente c) Methacrylsäure umfasst oder aus Methacrylsäure besteht.

6. Copolymer nach einem der vorhergehenden Ansprüche, wobei die Komponente d) ausgewählt ist unter primären Amiden α,β-ethylenisch ungesättigter Monocarbonsäuren, N-Vinylamiden gesättigter Monocarbonsäuren, N-Vinyllactamen, N-Alkyl- und N,N-Dialkylamiden α,β-ethylenisch ungesättigter Monocarbonsäuren und Mischungen davon.

7. Copolymer nach einem der vorhergehenden Ansprüche, das zusätzlich wenigstens ein weiteres Monomer e) einpolymerisiert enthält, das ausgewählt ist unter von Komponente a) verschiedenen Estern α,β-ethylenisch ungesättigter Mono- und Dicarbonsäuren mit C₁-C₃₀-Alkanolen, ungesättigten C₈-C₃₀-Fettalkoholen und C₂-C₃₀-Alkandiolen, Amiden α,β-ethylenisch ungesättigter Mono- und Dicarbonsäuren mit C₂-C₃₀-Aminoalkoholen, die eine primäre oder sekundäre Aminogruppe aufweisen, Estern von Vinylalkohol und Allylalkohol mit C₁-C₃₀-Monocarbonsäuren, Vinylethern, Vinylaromaten, Vinylhalogeniden, Vinylidenhalogeniden, C₂-C₈-Monoolefinen, nicht aromatischen Kohlenwasserstoffen mit mindestens zwei konjugierten Doppelbindungen und Mischungen davon.

8. Kosmetisches oder pharmazeutisches Mittel, enthaltend
A) wenigstens ein ampholytisches Copolymer, wie in einem der Ansprüche 1 bis 7 definiert, und
B) wenigstens einen kosmetisch akzeptablen Träger.

9. Mittel nach Anspruch 8, wobei die Komponente B) ausgewählt ist unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen
und Mischungen davon.

10. Mittel nach einem der Ansprüche 8 oder 9 in Form eines Sprays, Gels, Schaums, einer Mousse, Salbe, Creme, Emulsion, Suspension, Lotion, Milch oder Paste.

11. Verwendung eines Copolymers, wie in einem der Ansprüche 1 bis 7 definiert, in Hautreinigungsmitteln, Mitteln zur Pflege und zum Schutz der Haut, Nagelpflegemitteln, Zubereitungen für die dekorative Kosmetik und Haarbehandlungsmitteln.

12. Verwendung nach Anspruch 11 in Haarbehandlungsmitteln als Festiger und/oder als Conditioner.

13. Verwendung nach Anspruch 12, wobei das Mittel in Form eines Haargels, Shampoos, Schaumfestigers, Haarwassers, Haarsprays oder Haarschaums oder einer Mousse vorliegt.

14. Verwendung nach einem der Ansprüche 11 bis 13, wobei das Mittel 60 Gew.-% oder weniger VOC enthält.

15. Verwendung eines Copolymers, wie in einem der Ansprüche 1 bis 7 definiert, als Hilfsmittel in der Pharmazie, bevorzugt als oder in Beschichtungsmittel(n) für feste Arzneiformen, zur Modifizierung rheologischer Eigenschaften, als oberflächenaktive Verbindung, als oder in Klebemittel(n) sowie als oder in Beschichtungsmittel(n) für die Textil-, Papier-, Druck- und Lederindustrie.

## Claims

1. An ampholytic copolymer A which has a molar excess of cationogenic/cationic groups compared to anionogenic/anionic groups and is obtainable by free-radical polymerization of
a) at least one α,β-ethylenically unsaturated monomer of the general formula I 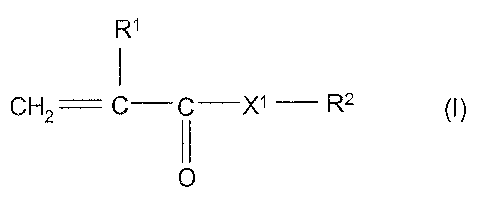 in which
R¹ is hydrogen or C₁-C₈-alkyl,
X¹ is O or NR³, where R³ is hydrogen, alkyl, cycloalkyl, aryl or hetaryl,
R² is branched C₃-C₅-alkyl,
b) at least one compound with a free-radically polymerizable, α,β-ethylenically unsaturated double bond and at least one cationogenic and/or cationic group per molecule, with the proviso that at least some of the compounds b) have at least one quaternary nitrogen atom,
c) at least one compound with a free-radically polymerizable, α,β-ethylenically unsaturated double bond and at least one anionogenic and/or anionic group per molecule and
d) if appropriate at least one amide-group-containing monomer which is chosen from α,β-ethylenically unsaturated amide-group-containing compounds of the general formula II 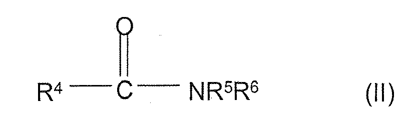 where
one of the radicals R⁴ to R⁶ is a group of the formula CH₂=CR⁷ -where R⁷ = H or C₁-C₄-alkyl and the other radicals R⁴ to R⁶, independently of one another, are H, alkyl, cycloalkyl, heterocycloalkyl, aryl or hetaryl,
where R⁴ and R⁵, together with the amide group to which they are bonded, may also be a lactam with 5 to 8 ring atoms,
where R⁵ and R⁶, together with the nitrogen atom to which they are bonded, may also be a five-to seven-membered heterocycle.

2. The copolymer according to claim 1, where component a) comprises at least one monomer of the formula I in which R² is tert-butyl.

3. The copolymer according to one of the preceding claims, where component a) comprises tert-butyl acrylate or consists of tert-butyl acrylate.

4. The copolymer according to one of the preceding claims, where component b) is chosen from esters of α,β-ethylenically unsaturated mono- and dicarboxylic acids with amino alcohols which may be mono- or dialkylated on the amine nitrogen, amides of α,β-ethylenically unsaturated mono- and dicarboxylic acids with diamines which have at least one primary or secondary amino group, N,N-diallylamine, N,N-diallyl-N-alkylamines and derivatives thereof, vinyl- and allyl-substituted nitrogen heterocycles, vinyl- and allyl-substituted heteroaromatic compounds, the quaternization products of these monomers and mixtures thereof.

5. The copolymer according to one of the preceding claims, where component c) comprises methacrylic acid or consists of methacrylic acid.

6. The copolymer according to one of the preceding claims, where component d) is chosen from primary amides of α,β-ethylenically unsaturated monocarboxylic acids, N-vinylamides of saturated monocarboxylic acids, N-vinyllactams, N-alkyl- and N,N-dialkylamides of α-β-ethylenically unsaturated monocarboxylic acids and mixtures thereof.

7. The copolymer according to one of the preceding claims which additionally comprises, in copolymerized form, at least one further monomer e) which is chosen from esters of α,β-ethylenically unsaturated mono- and dicarboxylic acids with C₁-C₃₀-alkanols, unsaturated C₈-C₃₀ fatty alcohols and C₂-C₃₀-alkanediols different from component a), amides of α,β-ethylenically unsaturated mono- and dicarboxylic acids with C₂-C₃₀-amino alcohols which have a primary or secondary amino group, esters of vinyl alcohol and allyl alcohol with C₁-C₃₀-monocarboxylic acids, vinyl ethers, vinylaromatics, vinyl halides, vinylidene halides, C₂-C₈-monoolefins, nonaromatic hydrocarbons with at least two conjugated double bonds and mixtures thereof.

8. A cosmetic or pharmaceutical composition comprising
A) at least one ampholytic copolymer as defined in one of claims 1 to 7 and
B) at least one cosmetically acceptable carrier.

9. The composition according to claim 8, where component B) is chosen from
i) water,
ii) water-miscible organic solvents, preferably C₂-C₄-alkanols, in particular ethanol,
iii) oils, fats, waxes,
iv) esters of C₆-C₃₀-monocarboxylic acids with mono-, di- or trihydric alcohols different from iii),
v) saturated acyclic and cyclic hydrocarbons,
vi) fatty acids,
vii) fatty alcohols,
viii) propellent gases
and mixtures thereof.

10. The composition according to claim 8 or 9 in the form of a spray, gel, foam, mousse, ointment, cream, emulsion, suspension, lotion, milk or paste.

11. The use of a copolymer as defined in one of claims 1 to 7 in skin-cleansing compositions, compositions for the care and protection of the skin, nailcare compositions, preparations for decorative cosmetics and hair-treatment compositions.

12. The use according to claim 11 in hair-treatment compositions as setting agent and/or as conditioner.

13. The use according to claim 12, where the agent is in the form of a hair gel, shampoo, setting foam, hair tonic, hairspray or hair foam or a mousse.

14. The use according to one of claims 11 to 13, where the composition comprises 60% by weight or less of VOC.

15. The use of a copolymer as defined in one of claims 1 to 7 as auxiliary in pharmacy, preferably as or in coating(s) for solid drug forms, for modifying rheological properties, as surface-active compound, as or in adhesive(s), and as or in coating(s) for the textile, paper, printing and leather industries.

## Revendications

1. Copolymère A ampholyte, qui présente un excès molaire en groupes cationogènes/cationiques par rapport aux groupes anionogènes/anioniques et qui peut être obtenu par polymérisation radicalaire de
a) au moins un monomère à insaturation α,β-éthylénique, de formule générale I 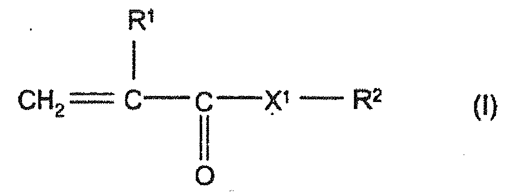 dans laquelle
R¹ représente un atome d'hydrogène ou un groupe alkyle en C₁-C₈,
X¹ représente O ou NR³, R³ représentant un atome d'hydrogène, un groupe alkyle, cycloalkyle, aryle ou hétéroaryle,
R² représente un groupe alkyle en C₃-C₅,
b) représente au moins un composé comportant une double liaison à insaturation α,β-éthylénique, polymérisable par voie radicalaire, et au moins un groupe cationogène et/ou un groupe cationique par molécule, étant entendu qu'au moins une partie des composés b) comporte au moins un atome d'azote quaternaire,
c) au moins un composé comportant une double liaison à insaturation α,β-éthylénique, polymérisable par voie radicalaire, et au moins un groupe anionogène et/ou un groupe anionique par molécule et
d) éventuellement au moins un monomère contenant des groupes amido, qui est choisi parmi des composés contenant des groupes amido, à insaturation α,β-éthylénique, de formule générale II 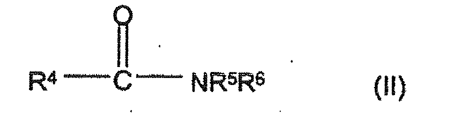 dans laquelle
l'un des radicaux R⁴ à R⁶ représente un groupe de formule CH₂=CR⁷- où R⁷ = H ou un groupe alkyle en C₁-C₄ et les autres radicaux R⁴ à R⁶ représentent, chacun indépendamment, H, un groupe alkyle, cycloalkyle, hétérocycloalkyle, aryle ou hétéroaryle,
R⁴ et R⁵ pouvant également représenter ensemble, avec le groupe amido auquel ils sont liés, un lactame ayant de 5 à 8 atomes formant le cycle,
R⁵ et R⁶ pouvant également représenter, avec l'atome d'azote auquel ils sont liés, un hétérocycle à 5-7 chaînons.

2. Copolymère selon la revendication 1, dans lequel le composant a) comprend au moins un monomère de formule I dans lequel R² représente le groupe tert-butyle.

3. Copolymère selon l'une quelconque des revendications précédentes, dans lequel le composant a) comprend de l'acrylate de tert-butyle ou consiste en acrylate de tert-butyle.

4. Copolymère selon l'une quelconque des revendications précédentes, dans lequel le composant b) est choisi parmi des esters d'acides monocarboxyliques et d'acides dicarboxyliques à insaturation α,β-éthylénique avec des aminoalcools, qui peuvent être mono- ou dialkylés sur l'atome d'azote en fonction amine, des amides d'acides monocarboxyliques et d'acides dicarboxyliques à insaturation α,β-éthylénique, qui comportent au moins un groupe amino primaire ou secondaire, la N,N-diallylamine, des N,N-diallyl-N-alkylamines et leurs dérivés, des hétérocycles azotés substitués par vinyle et allyle, des composés hétéroaromatiques substitués par vinyle et allyle, les produits de quaternisation de ces monomères et des mélanges de ceux-ci.

5. Copolymère selon l'une quelconque des revendications précédentes, dans lequel le composant c) comprend de l'acide méthacrylique ou consiste en acide méthacrylique.

6. Copolymère selon l'une quelconque des revendications précédentes, dans lequel le composant d) est choisi parmi des amides primaires d'acides monocarboxyliques à insaturation α,β-éthylénique, des N-vinylamides d'acides monocarboxyliques saturés, des N-vinyllactames, des N-alkyl- et N,N-dialkylamides d'acides monocarboxyliques à insaturation α,β-éthylénique et des mélanges de ceux-ci.

7. Copolymère selon l'une quelconque des revendications précédentes, qui en outre contient au moins un autre monomère e) incorporé par polymérisation, qui est choisi parmi des esters, différents du composant a), d'acides monocarboxyliques et d'acides dicarboxyliques à insaturation α,β-éthylénique avec des alcanols en C₁-C₃₀, des alcools gras insaturés en C₈-C₃₀ et des alcanediols en C₂-C₃₀, des amides d'acides monocarboxyliques et d'acides dicarboxyliques à insaturation α,β-éthylénique avec des aminoalcools en C₂-C₃₀ qui comportent un groupe amino primaire ou secondaire, des esters d'alcool vinylique et d'alcool allylique avec des acides monocarboxyliques en C₁-C₃₀, des éthers vinyliques, des composés vinylaromatiques, des halogénures de vinyle, des halogénures de vinylidène, des mono-oléfines en C₂-C₈, des hydrocarbures non aromatiques comportant au moins deux doubles liaisons conjuguées, et des mélanges de ceux-ci.

8. Composition pharmaceutique ou cosmétique, contenant
A) au moins un copolymère ampholyte, tel que défini dans l'une quelconque des revendications 1 à 7, et
B) au moins un véhicule cosmétiquement acceptable.

9. Composition selon la revendication 8, dans laquelle le composant B) est choisi parmi
i) l'eau,
ii) des solvants organiques miscibles à l'eau, de préférence des alcanols en C₂-C₄, en particulier l'éthanol,
iii) des huiles, des graisses, des cires,
iv) des esters, différents de iii), d'acides monocarboxyliques en C₆-C₃₀, avec des alcools mono-, di- ou trihydriques,
(v) des hydrocarbures acycliques saturés et hydrocarbures cycliques saturés,
vi) des acides gras,
vii) des alcools gras,
viii) des gaz propulseurs
et des mélanges de ceux-ci.

10. Composition selon la revendication 8 ou 9, sous forme d'une composition à pulvériser en aérosol, d'un gel, d'un produit moussant, d'une mousse, pommade, crème, émulsion, suspension, lotion, d'un lait ou d'une pâte.

11. Utilisation d'un copolymère, tel que défini dans l'une quelconque des revendications 1 à 7, dans des produits de nettoyage de la peau, des produits pour le soin et la protection de la peau, des produits pour le soin des ongles, des préparations pour la cosmétique de maquillage et des produits de traitement capillaire.

12. Utilisation selon la revendication 11, dans des produits de traitement capillaire, en tant que fixateurs et/ou conditionneurs.

13. Utilisation selon la revendication 12, dans laquelle le produit se trouve sous forme d'un gel capillaire, d'un shampooing, d'un fixateur en mousse, d'une lotion capillaire, d'une laque pour cheveux ou d'un produit capillaire moussant ou d'une mousse.

14. Utilisation selon l'une quelconque des revendications 11 à 13, dans laquelle la composition contient 60 % en poids ou moins de COV (composés organiques volatils).

15. Utilisation d'un copolymère, tel que défini dans l'une quelconque des revendications 1 à 7, en tant qu'adjuvant en pharmacie, de préférence en tant que ou dans des composition(s) d'enrobage pour des formes pharmaceutiques solides, pour la modification de propriétés rhéologiques, en tant que composé tensioactif, en tant qu'adhésif ou dans des adhésifs ainsi qu'en tant que ou dans des composition(s) de revêtement pour l'industrie textile, du papier, de l'impression et du cuir.
